# EUROPEAN PATENT APPLICATION

(11) **EP 2 105 118 A1**
(43) Date of publication of application: **30.09.2009**
(21) Application number: 09004123.7
(22) Date of filing: 23.03.2009
(51) Int. Cl.: A61H 33/12

(54) **Mist generator**

(30) Priority: 26.03.2008 JP 2008079866
(71) Applicant: Panasonic Electric Works Co., Ltd., Kadoma-shi Osaka 571-8686 (JP)
(72) Inventor: Watanabe, Shunichi, Kadoma-shi Osaka 571-8686 (JP); Takeuchi, Toshihiro, Kadoma-shi Osaka 571-8686 (JP); Funatsu, Keiko, Kadoma-shi Osaka 571-8686 (JP)
(74) Representative: Schatt, Markus F.

(57) **Abstract**

A mist generator (10) including a warm mist generation mechanism (40) and a movable protective device (21). The warm mist generation mechanism generates warm mist and includes a warm mist nozzle (18) for spraying the generated warm mist in a spraying direction. The movable protective device prevents a hand of a user from touching the warm mist nozzle. The protective device is movable between a first position, which allows the warm mist to be sprayed from the warm mist nozzle toward the user, and a second position, which covers at least an upper part of the warm mist nozzle.

## Description

### BACKGROUND OF THE INVENTION

The present invention relates to a mist generator for stimulating the skin of a user with mist.

In the prior art, a mist generator is known as a cosmetic device that produces a cosmetic effect on a user's skin. The mist generator sprays a mist of liquid such as water toward a user. Japanese Laid-Open Patent Publication No. 10-15049 (hereinafter referred to as '049 publication) discloses a facial cosmetic device (mist generator) that sprays from a spray nozzle warm steam, which is generated by heating liquid, and a cool mist, the temperature of which is lower than the warm steam, toward the user's skin. Japanese Utility Model Publication No. 6-9654 (hereinafter referred to as '654 publication) proposes a mist generator for spraying from a spray nozzle a mixed mist, in which warm mist generated by a heater is mixed with cool mist generated by an ultrasonic vibrator, toward the user. The end of the spray nozzle is narrowed.

### SUMMARY OF THE INVENTION

With each of the mist generators of the '049 publication and the '654 publication, the angle of the nozzle is adjusted to adjust the spraying direction of the mist in accordance with the position of the user's face of the user. However, the movable range of the nozzle is relatively narrow, and the flow of mist cannot be greatly separated from the user when adjusting the nozzle. Furthermore, the '049 publication and the '654 publication do not take into consideration the possibility that the user may directly touch the warm mist, which is relatively hot immediately after being sprayed out from the nozzle, or the nozzle, which is relatively hot.

The present invention is directed to a mist generator having a structure for preventing a user from touching a nozzle that sprays warm mist.

One aspect of the present invention provides a mist generator including a warm mist generation mechanism and a movable protective device. The warm mist generation mechanism generates warm mist and includes a warm mist nozzle for spraying the generated warm mist in a spraying direction. The movable protective device prevents a hand of a user from touching the warm mist nozzle. The protective device is movable between a first position, which allows the warm mist to be sprayed from the warm mist nozzle toward the user, and a second position, which covers at least an upper part of the warm mist nozzle.

Other aspects and advantages of the present invention will become apparent from the following description, taken in conjunction with the accompanying drawings, illustrating by example the principles of the invention.

### BRIEF DESCRIPTION OF THE DRAWINGS

The invention, together with objects and advantages thereof, may best be understood by reference to the following description of the presently preferred embodiments together with the accompanying drawings in which:
Fig. 1 is a perspective view showing a cosmetic device in one embodiment;
Fig. 2(a) is a front view showing the cosmetic device of Fig. 1;
Fig. 2(b) is a side view showing the cosmetic device of Fig. 1;
Fig. 2(c) is a front view showing the cosmetic device with a main body lid closed;
Fig. 2(d) is a side view showing the cosmetic device of Fig. 2(c);
Fig. 3 is a cross-sectional view taken along line 3-3 of the cosmetic device of Fig. 2(c);
Fig. 4 is a partial exploded perspective view showing the main body lid;
Fig. 5 is an exploded perspective view showing a cap;
Figs. 6(a) and 6(b) are cross-sectional views of a cap and a nozzle cover;
Fig. 7 is a cross-sectional view taken along line 7-7 shown in Fig. 2(a);
Fig. 8 is a cross-sectional view taken along line 8-8 shown in Fig. 7;
Fig. 9(a) is a cross-sectional view taken along line 9-9 shown in Fig. 2(a);
Fig. 9(b) is an enlarged cross-sectional view showing a cool mist nozzle (cool mist spray port);
Fig. 10 is a partial perspective view showing a drain outlet;
Fig. 11 is a partial exploded perspective view showing a drainage mechanism;
Figs. 12(a) and 12(b) are cross-sectional views taken along line 12-12 of the drainage mechanism of Fig. 10 in a closed state and an open state, respectively;
Fig. 13(a) is a cross-sectional view taken along line 13a-13a shown in Figs. 2(b) and 3;
Fig. 13(b) is a cross-sectional view taken along line 13b-13b of Fig. 13(a);
Fig. 14 is an exploded perspective view showing a fragrance container removable mechanism;
Figs. 15(a), 15(b), and 15(c) are perspective views of a fragrance container;
Figs. 16(a), 16(b), and 16(c) are respectively plan view, side view, and cross-sectional view showing the fragrance plate;
Fig. 17 is a block diagram of the cosmetic device of Fig. 1;
Figs. 18(a) and 18(b) are respectively a side view and a plan view of the cosmetic device in the usage state;
Fig. 19 is an exploded perspective view showing the cap of a first modification;
Figs. 20(a) and 20(b) are cross-sectional views of the cap of second and third modifications;
Figs. 21(a) and 21(b) are cross-sectional views of the cap of fourth and fifth modifications;
Fig. 22 is a cross-sectional view showing a fragrance container (lid member) of another modification; and
Figs. 23(a) and 23(b) are cross-sectional views of a fragrance plate of a further modification.

### DETAILED DESCRIPTION OF THE PREFERRED EMBODIMENTS

A cosmetic device according to one embodiment of the present invention will be described.

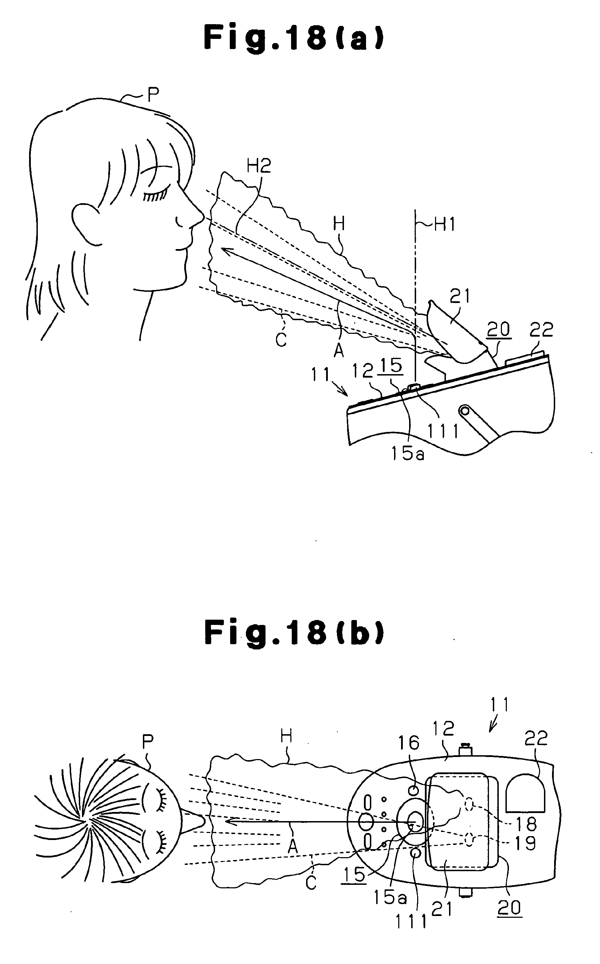

In the following description, the state in which a user P (shown in Fig. 18) uses a cosmetic device will be used as the reference of frame for the terms "front", "rear", "up", "down", "left", and "right."

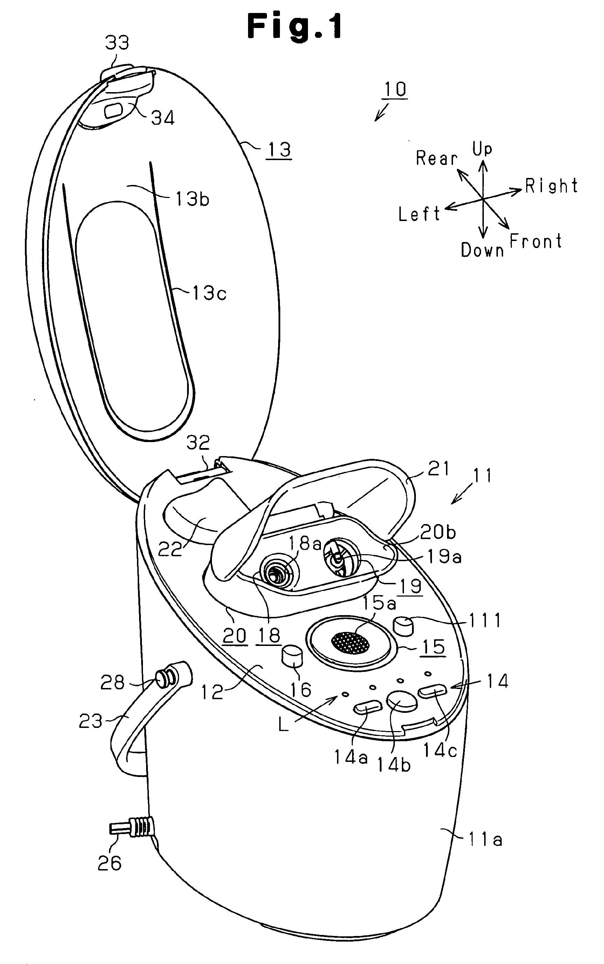

As illustrated in Fig. 1, a cosmetic device 10, which is one example of a mist generator, has a main body 11 including an elliptic cylindrical housing 11a and a top surface 12 that covers an inclined upper end opening of the housing 11a. A main body lid 13 is pivotally attached to the main body 11 by a hinge 32. The main body lid 13 has an elliptical top lid surface 13b. The top lid surface 13b has a recessed inner surface so that the inner side of the main body lid 13 is narrow.

A warm mist nozzle 18, which includes a warm mist spray port 18a for spraying warm mist H, and a cool mist nozzle 19, which includes a cool mist spray port 19a for spraying cool mist C, are arranged next to each other at the central part of the top surface 12. Fig. 18 shows the mists H and C sprayed from the mist spray ports 18a and 19a.

The mist nozzles 18 and 19, excluding the spray ports 18a and 19a, are covered by a nozzle cover (spraying direction adjustment means) 20. The nozzle cover 20 is dome-shaped although its shape is not limited. The nozzle cover 20 is attached to the main body 11 in a manner pivotal in upward and downward directions (i.e., vertical direction). The spraying directions of the mists H and C sprayed from the mist nozzles 18 and 19 may be adjusted in the vertical direction by pivoting the nozzle cover 20 in the vertical direction.

The mists H and C are sprayed in directions extending along the axes of the corresponding mist spray ports 18a and 19a (line H2 of Fig. 18). For example, the mist nozzles 18 and 19 may each be slightly tilted inward so that the mists H and C overlap each other in the spraying direction. The tilt of the mist nozzles 18 and 19 may be set such that the corresponding mists H and C are sprayed toward the center (front) of the user P when the user P is positioned in front of the cosmetic device 10 (see Fig. 18(b)). In the illustrated example, the warm mist nozzle 18 is arranged on the left side, and the cool mist nozzle 19 is arranged on the right side.

The nozzle cover 20 is capable of adjusting the spraying direction H2 of the mists H and C between upper limit direction and lower limit direction. For example, when the nozzle cover 20 is located at an upper limit position, the mists H and C may be sprayed above the upper side of the user P (upper limit direction). When the nozzle cover 20 is located at a lower limit position, the mists H and C may be sprayed in lower limit direction that is downward the upper limit direction, for example, the mists H and C may be sprayed horizontally with respect to the surface on which the cosmetic device 10 is set. The lower limit position of the nozzle cover 20 is set such that the mists H and C do not strike the top surface 12.

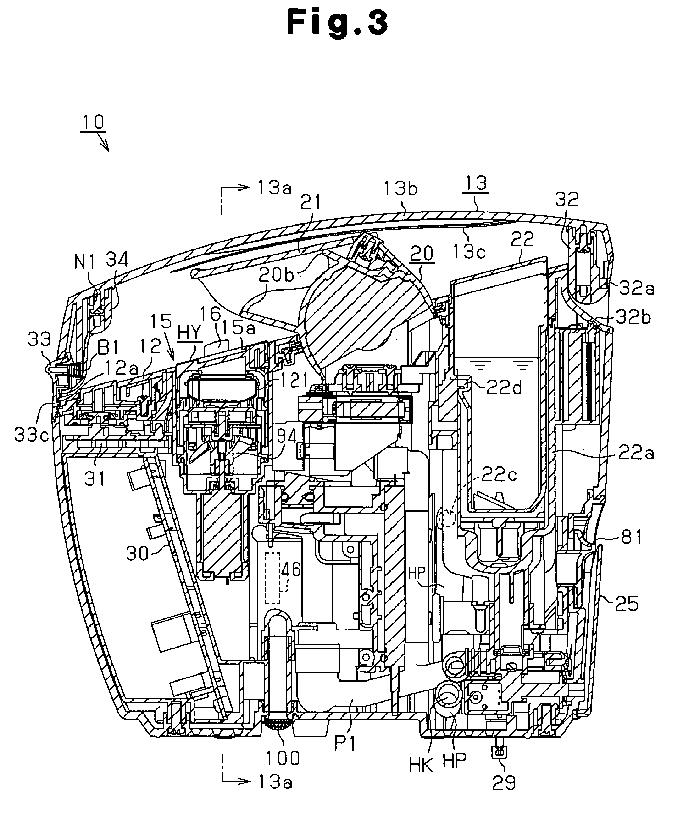

A mist guide 20b is arranged on the nozzle cover 20 (see Fig. 3). The mist guide 20b may be a wall extending from the periphery of the mist nozzles 18 and 19. The mist guide 20b includes a relatively short wall portion, which is on the upper side of the mist nozzles 18 and 19, and a relatively short wall portion, which is on the lower side of the mist nozzles 18 and 19. The mist guide 20b guides the mists H and C in a direction extending along the axes of the mist spray ports 18a and 19a.

A protective device, or cap 21, pivotal in the vertical direction is attached to the nozzle cover 20.

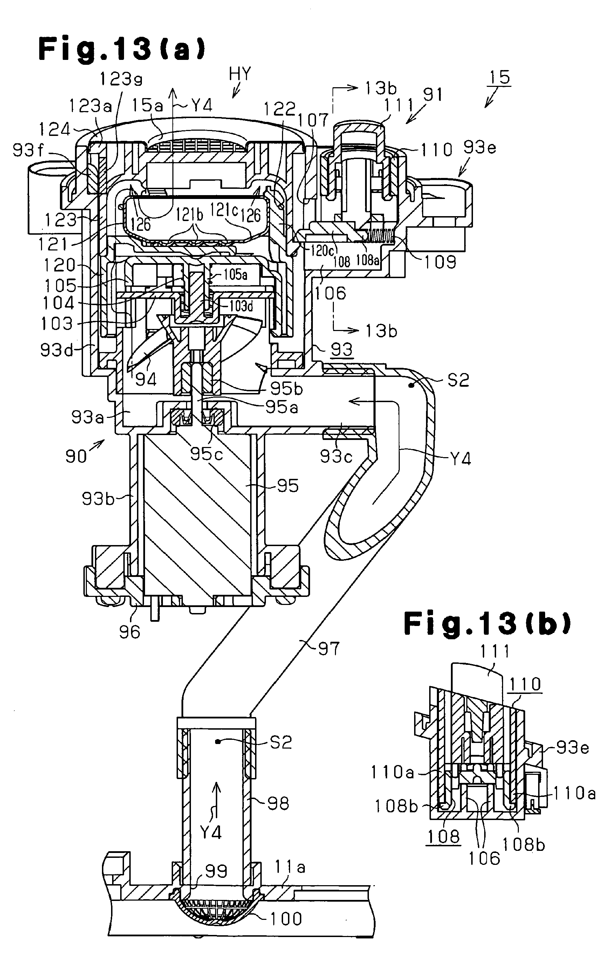

A fragrance generation unit 15 of the cosmetic device 10 volatilizes an aromatic substance to generate a fragrance. With reference to Figs. 3 and 13, the fragrance generation unit 15 includes a fragrance container HY for accommodating a fragrance plate (holding means) 121, which holds an aromatic substance. The fragrance container HY may be projected upward from the top surface 12 so that it may be lifted by operating a discharge button. This enables removal of the fragrance container HY from the fragrance generation unit 15. The fragrance container HY is removable in a direction intersecting the spraying direction H2 of the mists H and C. The fragrance generation unit 15 is separate from the mist nozzles 18 and 19 and arranged frontward from the mist nozzles 18 and 19 in the main body 11. The fragrance generated in the fragrance generation unit 15 is emitted upward through a fragrance emission port (fragrance emission means) 15a. The fragrance emission port 15a is arranged frontward from and near the mist spray ports 18a and 19a at the vicinity of the mist spray ports 18a and 19a. Thus, the fragrance emission port 15a is closer to the user than the mist spray ports 18a and 19a. The horizontal position of the fragrance emission port 15a may be between (preferably middle) the warm mist spray port 18a and the cool mist spray port 19a. In the illustrated example, the center of each mist spray port 18a and 19a and the center of the fragrance emission port 15a form an isosceles triangle. The fragrance emission port 15a may be formed below the mist spray ports 18a and 19a.

As shown in Fig. 3, the mist guide 20b of the nozzle cover 20 adjusted to the lower position is arranged immediately above part of the fragrance generation unit 15 (fragrance emission port 15a). If the nozzle cover 20 is adjusted to the lower position, even when trying to lift the fragrance container HY out of the fragrance generation unit 15, the fragrance container HY cannot be removed due to interference (contact) with the mist guide 20b. The fragrance container HY is removable only when the nozzle cover 20 is adjusted to the upper position.

The mist guide 20b partially, not completely, covers the fragrance emission port 15a even if the nozzle cover 20 is adjusted to the lower position. Thus, fragrance may be emitted toward the mists H and C from the fragrance emission port 15a even if the nozzle cover 20 is adjusted to the lower position.

A power button 16, which is arranged on the left side of the fragrance generation unit 15, may be a mechanical switch. A fragrance container discharge button (discharge operation member) 111 operated to remove the fragrance container HY is arranged on the right side of the fragrance generation unit 15. The fragrance container discharge button 111 is arranged at a position where the hand of the user P, who presses the fragrance container discharge button 111, would not touch the mists H and C. When the nozzle cover 20 is adjusted to the lower position, the mist guide 20b guides a flow of the mists H and C so as not to strike the fragrance container discharge button 111. The lower limit in the pivot range of the nozzle cover 20 is determined such that a flow of the mists H and C would not strike the fragrance container discharge button 111.

An operation unit 14 used by the user P to operate the cosmetic device 10 and a display L for showing the operation state of the cosmetic device 10 to the user are arranged on the front side of the fragrance generation unit 15. The display L may use a lighting pattern (lighting, flashing, non-lighting, etc.) formed by a plurality of light emitting bodies (e.g., LED). The operation unit 14 includes an operation control button 14b, an operation mode selection button 14a, and a fragrance control button 14c. The operation control button 14b is operated to start and stop the operation of the cosmetic device 10. The operation mode selection button 14a is operated to select the operation mode.

The cosmetic device 10 is operable in a plurality of operation modes. The cosmetic device 10 switches the warm mist H and the cool mist C sprayed to the user P in predetermined time intervals in accordance with the operation mode. The operation mode includes a clear skin course, tone and firmness course, sebum care course, and the like, and is optimized in terms of cosmetic effects on the skin of the user P. The spraying periods for the warm mist H and the cool mist C and the number of times switching is performed is set for each operation mode. The operation mode of the cosmetic device 10 is sequentially switched whenever the operation mode selection button 14a is pressed. The cosmetic device 10 does not have an operation mode in which only the cool mist C is sprayed.

The fragrance control button 14c is used to adjust the intensity of the fragrance (volatilization volume, concentration of aromatic substance) emitted from the fragrance generation unit 15. The intensity of the fragrance sequentially switches from "weak" → "intermediate"
→ "strong" → "OFF (no fragrance)" whenever the fragrance control button 14c is pressed. Each of the buttons 14a to 14c outputs an operation signal when pressed.

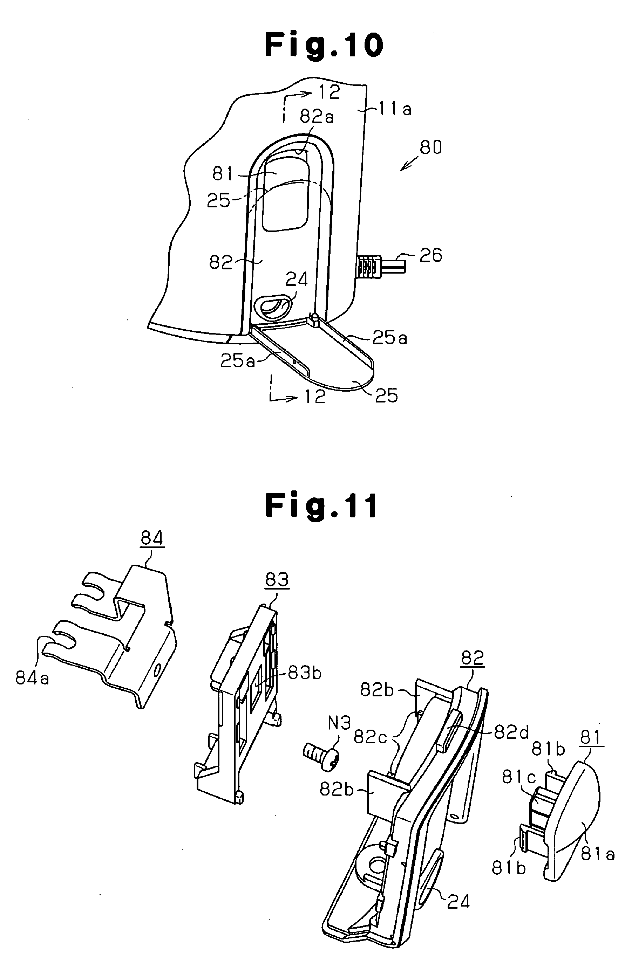

The main body 11 accommodates a tank 22 at the rear side of the nozzle cover 20. The tank 22 stores a predetermined amount (about 120 ml in the present embodiment) of liquid (water in the present embodiment). The liquid in the tank 22 is supplied to a warm mist generation mechanism (warm mist generation means) 40 and a cool mist generation mechanism (cool mist generation means) 60, which will be described later, and used to generate the mists H and C. As shown in Fig. 3, the main body 11 includes a tank holder 22a that opens in the top surface 12. The tank 22 is inserted into and removed out of the tank holder 22a in the vertical direction. When the main body lid 13 opens, the tank 22 may be pushed to pop it out of the tank holder 22a by a biasing means (not shown). This facilitates removal of the tank 22. When the tank 22 is pushed again, the tank 22 becomes fixed to a hook 22d. Thus the setting of the tank 22 is simple. An overflow hole 22c is formed in the lower part of the tank holder 22a to receive an overflow of excessive water supplied to the tank holder 22a. The overflow hole 22c is connected to a drain outlet 24 (Fig. 10) arranged on the rear surface of the main body 11 by a drainage pipe HP. The overflow hole 22c, the drain outlet 24, and the drainage pipe HP form a drainage passage HK for discharging overflow water out of the cosmetic device 10.

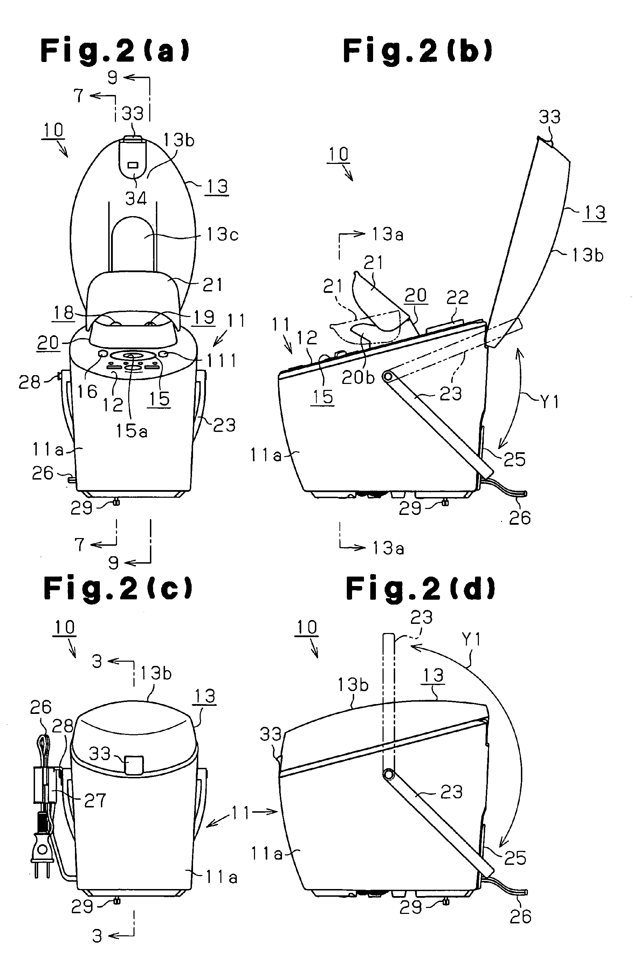

As shown in Figs. 1 and 2, an arcuate handle 23 is attached to the left and right sides of the main body 11. The handle 23 is pivotal with respect to the main body 11 (see arrow Y1 of Fig. 2(d)). The handle 23 is arranged at a carry position (double-dashed line of Fig. 2(d)) when the user P carries the cosmetic device 10, and the handle 23 is arranged at a lower position (solid line of Fig. 2(d)) when the user uses the cosmetic device 10. As shown in Fig. 2(b), when the main body lid 13 is open, the handle 23 cannot be pivoted from the lower position to the carry position due to contact with the main body lid 13. The user P needs to close the main body lid 13 in order to hold the handle 23 and carry the cosmetic device 10.

As shown in Figs. 2(b) and 10, the drain outlet 24 in the lower part on the rear surface of the main body 11 is covered by a pivotal drain outlet cover 25. The drain outlet cover 25 pivots about a lower end shaft (not shown). The water discharged from the drain outlet 24 is guided outside by a wall portion 25a of the drain outlet cover 25. A power supply cord 26 extends from the lower part of the main body 11. The power supply cord 26 is fixed between the housing 11a and a bushing holder (not shown), which is fastened by screws to the housing 11a. A cord band 27 attached to the power supply cord 26 is useful when bundling the power supply cords 26 (Fig. 2(c)). A projection 28 for hooking the hole of the cord band 27 is formed at the handle 23. The cord band 27 is hooked to the projection 28 with the power supply cords 26 bundled for storage when not using the cosmetic device 10.

An inclination detection switch 29 is biased to project out of the bottom surface of the main body 11. The inclination detection switch 29 detects a lift state and tilted state of the cosmetic device 10. If the cosmetic device 10 is placed on a flat surface such as a table surface, the inclination detection switch 29 is accommodated in the main body 11 due to the weight of the cosmetic device 10 and thus turned off (non-tilt detection). The inclination detection switch 29 is turned on (tilt detection) when the cosmetic device 10 is tilted or lifted.

The main body lid 13 will now be discussed with reference to Figs. 1 to 4.

When the main body lid 13 is in the open state, the top surface 12 is exposed. Thus, the cosmetic device 10 becomes operable. The main body lid 13 is maintained in the open state while using the cosmetic device 10. When the main body lid 13 is in the closed state, the entire top surface 12, that is, the mist nozzles 18 and 19, the nozzle cover 20, and the cap 21, the operation unit 14, the fragrance generation unit 15, and the tank 22 are accommodated in the space at the inner side of the main body lid 13. Foreign substances are prevented from collecting on the mist nozzles 18 and 19 when the cosmetic device 10 is not used by closing the main body lid 13. This is preferable for keeping the cosmetic device 10 hygienic. The spraying directions of the mists H and C is also avoided from being changed by the foreign substances collected on the mist nozzles 18 and 19. This prevents the usage feel of the cosmetic device 10 from being affected. In other words, each of the mists H and C are prevented from being sprayed in directions other than the predetermined direction.

As shown in Fig. 3, a magnet 32a is arranged on the hinge 32. A main body lid switch 32b of a lead switch type is arranged at a position corresponding to the magnet 32a in the main body 11. The main body lid switch 32b and the magnet 32a detect whether the main body lid 13 is in the open state.

A slide rib 13c having a step-form is arranged on the inner surface of the main body lid 13. The slide rib 13c contacts the cap 21 with a low slide resistance when closing the main body lid 13. This pivots the cap 21 downward.

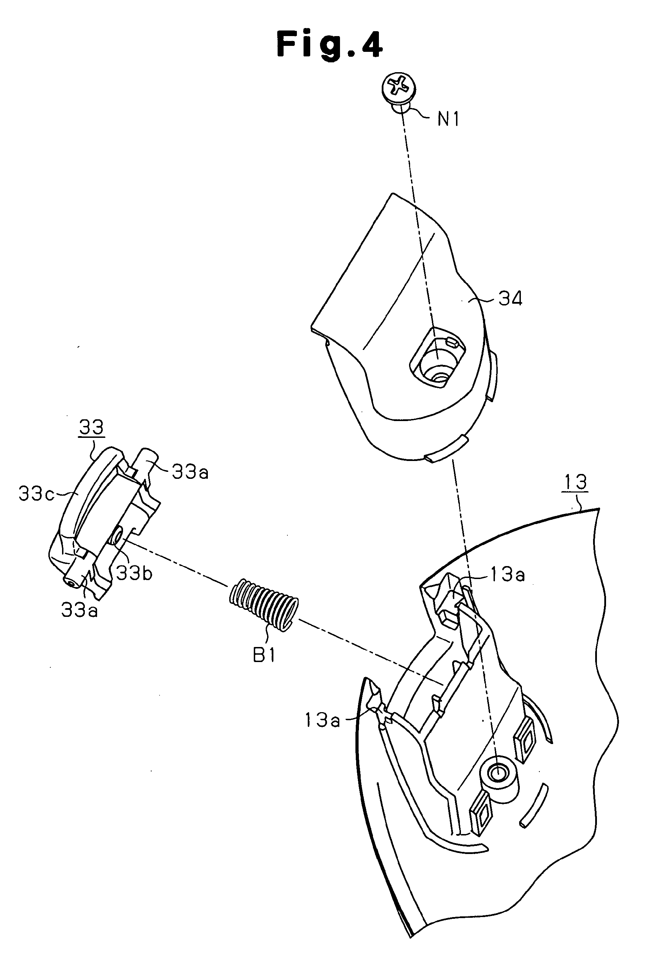

An open/close button 33 used to open or close the main body lid 13 is arranged on the front surface of the main body lid 13. As shown in Fig. 4, pivot shafts 33a project in opposite directions from the open/close button 33. The two pivot shafts 33a are each received by a recess 13a in the main body lid 13. The open/close button 33 pivots about the pivot shafts 33a. A button cover 34, which is fixed to the main body lid 13 by a screw N1, covers the open/close button 33. A spring B1 is held between the open/close button 33 and the main body lid 13. The spring B1 is positioned by a boss 33b of the open/close button 33. The spring B1 biases the open/close button 33 toward the front.

As shown in Figs. 3 and 4, a hook 33c is arranged at the distal end of the open/close button 33. The hook 33c engages a recess 12a formed in the top surface 12 of the main body 11 to keep the main body lid 13 closed. When the user P presses the open/close button 33, the open/close button 33 pivots about the pivot shaft 33a and disengages the hook 33c from the recess 12a to open the main body lid 13.

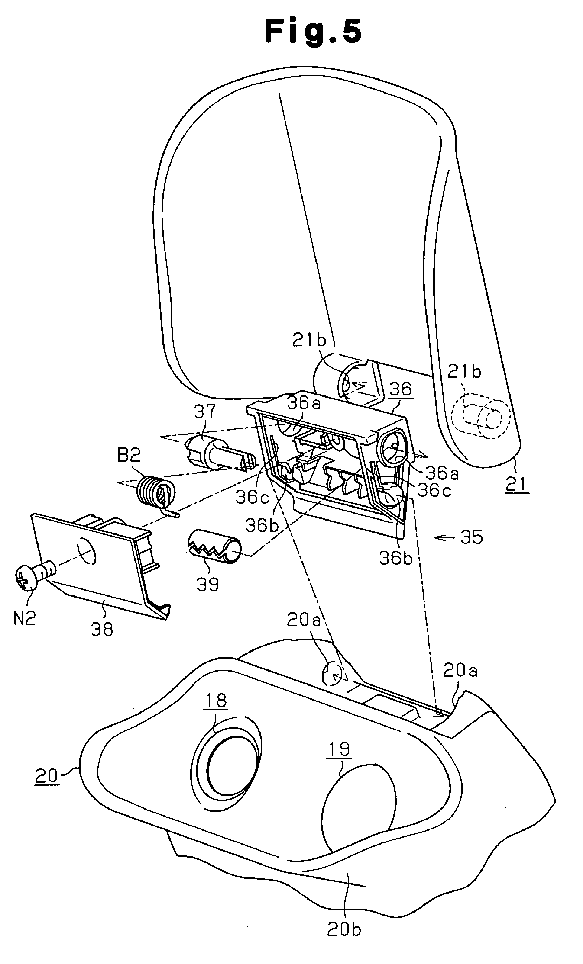

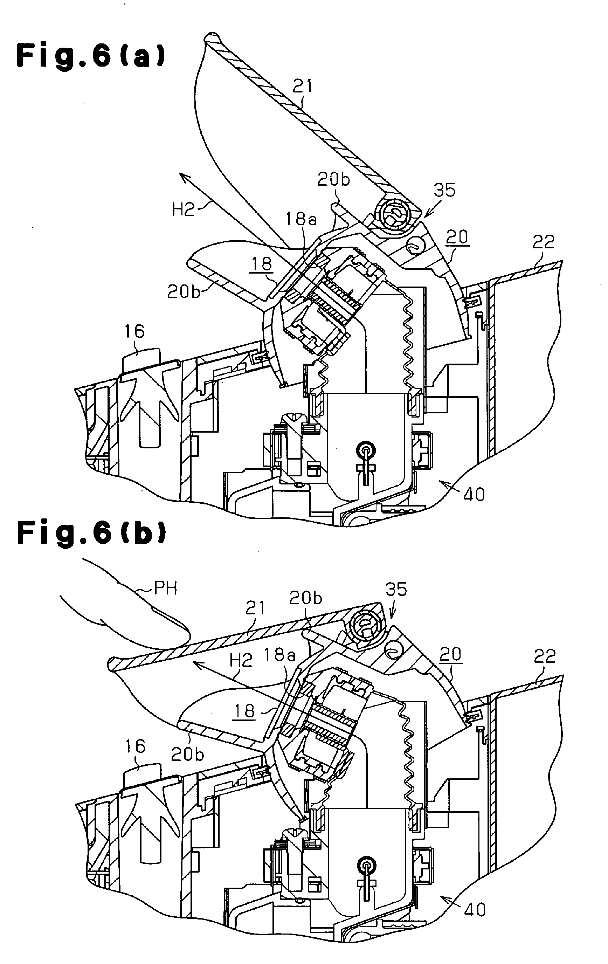

The movable cap 21 will now be discussed with reference to Figs. 5 and 6.

The cap 21 prevents the hand of the user P from touching each of the mist nozzles 18 and 19. The cap 21 is attached to the nozzle cover 20 by a nozzle hinge portion 35. In the illustrated embodiment, the cap 21 includes two downward wall portions and has the shape of a cap visor.

The cap 21 includes two pivot shafts 21b, which extend toward each other. A nozzle hinge 36 includes two shaft holes 36a, which corresponded to the two pivot shafts 21b of the cap 21. A spring shaft 37, which extends through one of the shaft holes 36a of the nozzle hinge 36, is fixed to one of the pivot shafts 21b of the cap 21. The pivot shaft 21b is fitted to a recess (not shown) formed in the spring shaft 37. A spring pin 39, which extends through the other shaft hole 36a of the nozzle hinge 36, is attached to the other pivot shaft 21b.

In this structure, the cap 21 is pivotal about the spring shaft 37 and the spring pin 39 between a spray position (shown with solid line of Fig. 2(b) and in Fig. 6(a)) and a protective position (shown with double-dashed line of Fig. 2(b) and in Fig. 6(b)). The spray position is a position suitable for spraying mist to the user P. The cap 21 does not block the spraying of the mists H and C when at the spray position. The protective position is a position where the spraying directions of the mists H and C are lowered. The cap 21 covers the upper side of the mist nozzles 18 and 19 when at the protective position. The spray position may be referred to as a first position in which the mist can be sprayed toward the user from the mist nozzle. The protective position may be referred to as a second position in which at least the upper part of the mist nozzle is covered.

As shown in Fig. 5, one end of a torsion coil spring B2 is fixed to the spring shaft 37. The other end of the torsion coil spring B2 is fixed to the nozzle hinge 36. A shaft cover 38, which protects components such as the spring shaft 37 and the torsion coil spring B2, is fixed to the nozzle hinge 36 by a screw N2. The biasing force of the torsion coil spring B2 is transmitted to the cap 21 via the spring shaft 37 so as to bias the cap 21 to the spray position.

A projection 36b is formed on the lower side of the shaft hole 36a at each of the left and right side surfaces of the nozzle hinge 36. A groove 36c is formed around each projection 36b excluding the portion coupled to the projection 16b. The groove 36c has a predetermined width, and communicates the inner side and the outer side of the nozzle hinge 36. When a predetermined load acts on each projection 36b, each projection 36b is resiliently moved inward into the nozzle hinge 36 as the coupled portion, which does not include the groove 36c deforms. When load no longer acts on each projection 36b, the resiliency of the material of the nozzle hinge 36 return the projections 36b to their original positions so as to project out of the side surfaces of the nozzle hinge 36. The groove 36c thus provides flexibility to the projection 36b.

The nozzle hinge portion 35 is fixed with The projections 36b engage the recesses 20a, which are formed at the upper part of the nozzle cover 20 and faced toward each other at the rear of the mist nozzles 18 and 19. The nozzle hinge portion 35 is detachable from the nozzle cover 20 due to the flexibility of the projections 36b. In other words, the cap 21 is detachably attached to the nozzle cover 20 by the nozzle hinge portion 35. Since the nozzle hinge portion 35 is arranged in a manner embedded in the nozzle cover 20, the cosmetic device 10 may be miniaturized.

The cap 21 pivots as it slides along the slide rib 13c in cooperation with the closing of the main body lid 13 and shifts to the protective position, at which is accommodated in the main body lid 13. The cosmetic device 10 can be miniaturized since the cap 21 is accommodated in the main body lid 13 when the main body lid 13 is closed.

The warm mist generation mechanism 40 incorporated in the main body 11 of the cosmetic device 10 will now be discussed.

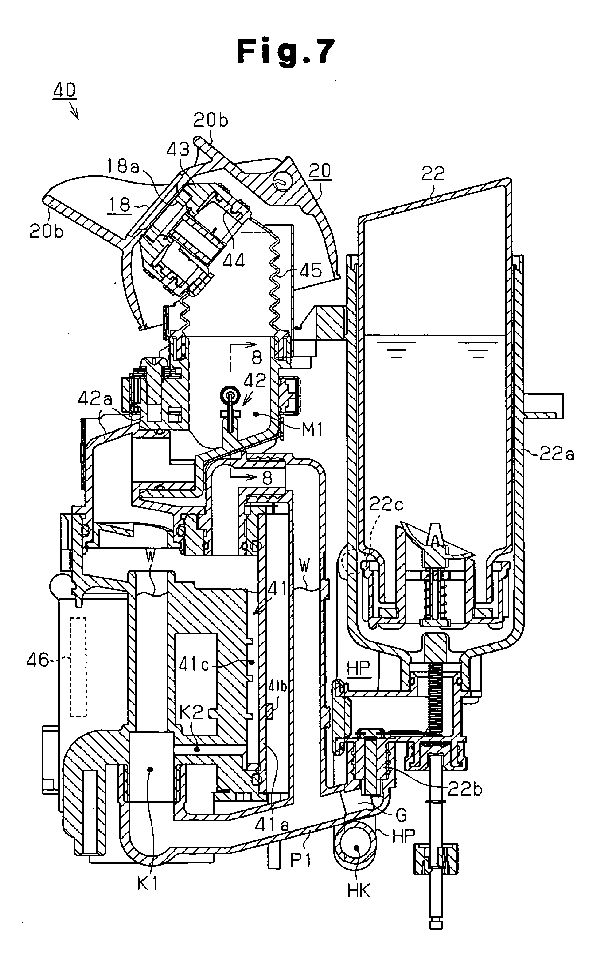

As shown in Fig. 7, the warm mist generation mechanism 40 mainly includes a boiler (warm mist generation unit) 41 for generating warm mist by heating water supplied from the tank 22, a discharge unit 42 for atomizing (ionizing) the warm mist, and the warm mist nozzle 18 for spraying the atomized warm mist.

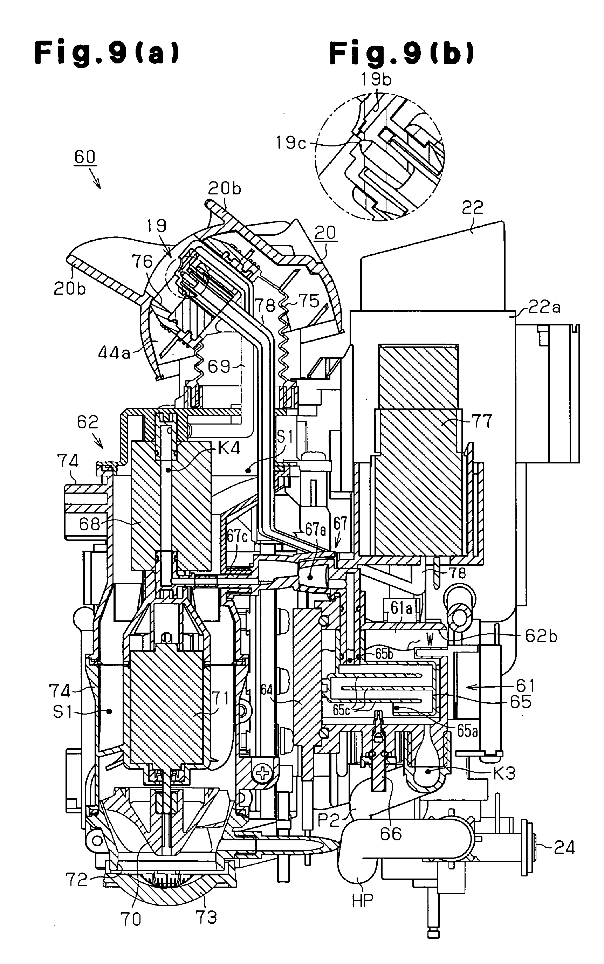

A connecting portion 22b arranged at the lower end of the tank holder 22a is coupled to a branched portion G. The branched portion G branches the water supplied from the tank 22 to the warm mist generation mechanism 40, the cool mist generation mechanism 60 (shown in Fig. 9), and the drain outlet 24 (shown in Fig. 10). The branched portion G is connected to the boiler 41 by a water supply pipe P1 made of elastic material (e.g., silicone rubber, fluorocarbon rubber) having heat resistance. The connecting portion 22b, the branched portion G, and the water supply pipe P1 form a water supply passage K1 for supplying water from the tank holder 22a to the boiler 41.

The boiler 41 includes a boiling chamber 41c equipped with a warm mist heater 41a for generating warm mist by heating the supplied water. The boiling chamber 41c is supplied with water from a water supply passage K2 branched to the side from the water supply passage K1. The boiling chamber 41c is formed to reduce the inner volume of the boiling chamber 41c while increasing the area of contact between the water and the warm mist heater 41a. The water level of the boiling chamber 41c is maintained at a predetermined water level W. The water level W is set so that the heated water is not sprayed from the warm mist nozzle 18 when the water is overly heated and boiled by the warm mist heater 41a. Further, the water level W is set so as to prevent a state in which there is no contact between the warm mist heater 41a of the boiling chamber 41c and water, that is, to prevent a boil dry state. The height (position) of the overflow hole 22c mentioned above is determined so as to discharge water that exceeds the water level W. The water level of the boiling chamber 41c is equal to the water level of the tank holder 22a. A heater thermistor 41b is fastened with a screw to a heat radiating surface (surface not contacting water) of the warm mist heater 41a.

A warm mist guiding member 42a for guiding the warm mist generated by the warm mist heater 41a to the warm mist nozzle 18 is connected at the upper side of the boiler 41. The warm mist guiding member 42a is connected to a lower end of an accordion member 45, which is accordion-shaped and formed from an elastic material (e.g., silicone rubber). An upper end of the accordion member 45 is connected to a nozzle holder 44, to which is attached a nozzle packing 43. The warm mist nozzle 18 is fixed to the nozzle packing 43. The warm mist guiding member 42a and the accordion member 45 form a warm mist path M1 for guiding the warm mist to the warm mist nozzle 18.

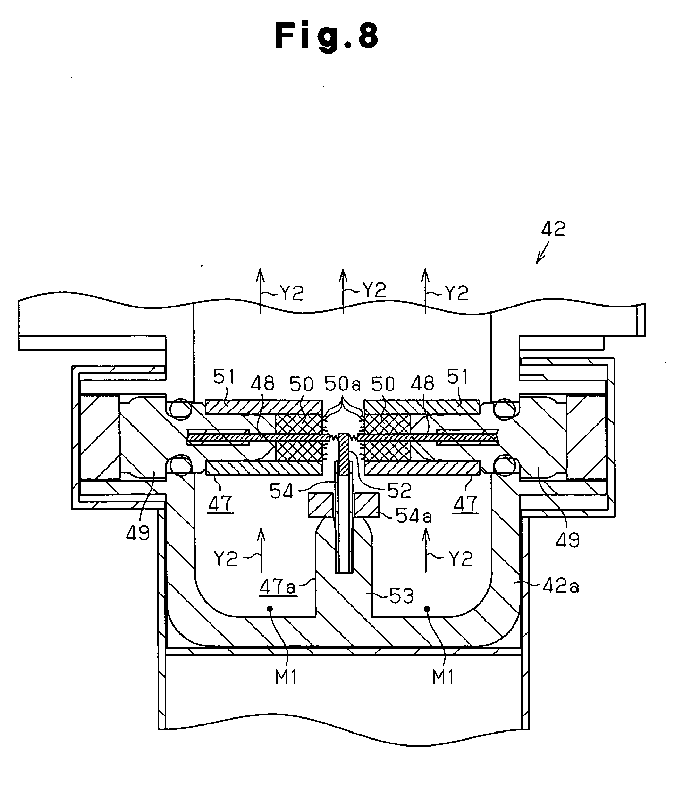

A discharge unit 42 is arranged on the warm mist path M1. As shown in Fig. 8, the discharge unit 42 includes a two electrodes 47 projecting to the inner side from the inner wall surface of the warm mist guiding member 42a, and an intermediate electrode 47a arranged at the midpoint of the pair of electrodes 47. Each electrode 47 includes a discharge needle 48. The lower side of Fig. 8 is the boiler 41 side, and the upper side of Fig. 8 is the warm mist nozzle 18 side. The warm mist generated in the boiler 41 flows in the Y2 direction.

Each discharge needle 48 is embedded and fixed in an electrode holder 49. The basal end of the electrode holder 49 is fixed to the inner wall of the warm mist guiding member 42a. Each discharge needle 48 is a needle having a diameter of 0.5 mm and formed from AgPd material or the like. Each discharge needle 48 is connected to a high voltage circuit board 46. High voltage is applied between the discharge needles 48. The discharge generated between the distal ends of the discharge needles 48 atomizes the warm mist.

A water absorbing body 50 is wrapped around the entire circumference of each discharge needle 48. The water absorbing body 50 is a fiber material such as nonwoven cloth. The distal end of the discharge needle 48 is exposed by a predetermined length (e.g., 0.5 mm to 1.0 mm) from the water absorbing body 50. Some of the fiber material forming the water absorbing body 50 falls apart into feather-like pieces, which projects out from the exposed surface of the water absorbing body 50 as microscopic fibers 50a. When the warm mist H collects and dew condenses at the distal end of the discharge needle 48 at where the ion generation electric field concentrates, it is known that the electric field distribution that generates between the discharge needles 48 is significantly disturbed, discharging becomes difficult, and the number of ions decreases. The water absorbing body 50 of the present embodiment absorbs water attached to the discharge needle 48 to stabilize the electric field distribution at each distal end of the discharge needle 48 and prevent disturbance of the electric field distribution.

Each electrode 47 includes an insulation body 51 for covering the water absorbing body 50 and the side surface of the electrode holder 49. The insulation body 51 is made of silicon rubber or fluorocarbon rubber, fluorocarbon resin, and the like.

The intermediate electrode 47a includes an intermediate electrode 52 held by an intermediate electrode holder 54 so as to be located at the middle between the discharge needles 48. The intermediate electrode holder 54 is press-fitted and fixed to a projection 53 formed in the warm mist guiding member 42a. The intermediate electrode 52 is, for example, a Pt rod having a diameter of 0.7 mm. The intermediate electrode holder 54 is made of stainless material (SUS 304 etc.). A water absorbing body 54a is arranged at the distal end portion of the intermediate electrode holder 54. The position of the intermediate electrode 52 is set such that the high voltage discharge between the discharge needles 48 is carried out only through the intermediate electrode 52, not through the intermediate electrode holder 54. A breakdown voltage necessary for high voltage discharge may be lowered by arranging the intermediate electrode 52 between the discharge needles 48, and discharging may be carried out at a lower voltage than when the intermediate electrode 52 is not arranged.

The cool mist generation mechanism 60 will now be discussed.

The main body 11 of the cosmetic device 10 also incorporates the cool mist generation mechanism 60. As shown in Fig. 9, the cool mist generation mechanism 60 mainly includes a heating processing unit 61 for sterilizing the water supplied from the tank 22, a cooling unit 62 for cooling the water sterilized in the heating processing unit 61, and the cool mist nozzle 19 for generating the cool mist from the cooled water by the Venturi effect and spraying the cool mist.

The branched portion G is connected to the heating processing unit 61 by a water supply pipe P2 made from an elastic material (e.g., silicone rubber). The branched portion G and the water supply pipe P2 form a water supply passage K3.

The heating processing unit 61 includes a reservoir chamber 61a for accumulating the water up to the water level W same as the tank holder 22a. The heating processing unit 61 includes a cool mist heater 64, which is fastened by a screw, so as to contact the water accumulated in the reservoir chamber 61a, and a damper section 65 for suppressing convection of the water heated by the cool mist heater 64. The damper section 65 includes a flow path meandering between a inlet port 65a formed on the lower side and a outlet portion 65b formed on the upper side. The length of the flow path of the damper section 65 becomes long due to meandering.

The damper section 65 will now be described in detail. A plurality of partition plates 65c alternately extending horizontally is arranged on the opposing inner walls of the damper section 65. The partition plates 65c form flow path partitioned into plural levels in the damper section 65. In the present embodiment, three partition plates 65c are arranged in the damper section 65 so as to partition the interior of the damper section 65 into four levels.

The distance (height) between the adjacent partition plates 65c is set so as to elongate the flow path formed in the damper section 65 and minimize increase in the path resistance that lowers the flow rate. The area of the inlet port 65a is minimized within a range that does not interfere with the flow of water. The inner volume of the damper section 65 is set such that it does not take too much time for the water flowing into the damper section 65 to reach a predetermined sterilization temperature (e.g., 80°C) when heated by the cool mist heater 64.

The sterilization temperature will be described. The sterilization temperature is set to a temperature sufficient for substantially eliminating bacteria, or sterilization, over a predetermined time (heating time). If the bacteria that is to be sterilized is Legionella, the elimination rate is substantially 100% by continuing the sterilization at 80°C for 30 seconds or longer. The time necessary for sterilization can be reduced by raising the sterilization temperature. However, vapor may be sprayed out of the cool mist nozzle 19 due to the boiling in the heating processing unit 61. Further, water may reversely flow into the tank holder 22a. Thus, the sterilizing temperature is set at the highest possible temperature at which the water does not boil in the heating processing unit 61. In the present embodiment, the sterilization temperature is set to 80°C and the heating time is set to 30 seconds.

A water temperature thermistor 66 for measuring the water temperature inside the damper section 65 is arranged in the reservoir chamber 61a. The water temperature thermistor 66 is arranged at a location where the water temperature tends to become the lowest inside the damper section 65. In the present embodiment, the water temperature thermistor 66 is arranged at the inlet port 65a of the damper section 65. A pressure opening port 62b communicated with the tank holder 22a by a tubular member (not shown) is formed above the water level W of the reservoir chamber 61a.

A gas-liquid exchange unit 67 is coupled to the outlet portion 65b of the damper section 65. The gas-liquid exchange unit 67 captures the bubbles contained in the water flowing out from the outlet portion 65b. This will be described in detail. The gas-liquid exchange unit 67 includes a gas-liquid exchange chamber 67a formed to a substantially cylindrical and extending in the horizontal direction. The outlet portion 65b of the damper section 65 is connected to the lower side on one end side (upstream side) of the gas-liquid exchange chamber 67a, and a outlet portion 67c from which the water flows out is formed at the lower side on the other end side (downstream side). In the gas-liquid exchange chamber 67a, the bubbles contained in the water supplied from the damper section 65 move to the upper side and are captured, and the bubbles and the water are exchanged. The bubbles generate or mix when the cool mist heater 64 heats water or when the water passes through the coupled part of each component. The inner volume of the gas-liquid exchange chamber 67a is set to be larger than the volume of the bubbles generated when using the cosmetic device 10.

A heat radiating member 68 includes a water supply passage K4. The outlet portion 67c of the gas-liquid exchange unit 67 is coupled to the lower part of the water supply passage K4. The heat radiating member 68 includes aluminum fins and the like, radiates the heat of the water supplied into the water supply passage K4 ,and cools the water. The water flows from the lower part (upstream side) of the water supply passage K4 to the upper part (downstream side) of the water supply passage K4. A water supply tube 69 is connected to the upper part (downstream side) of the water supply passage K4. The water supply tube 69 is connected to the cool mist nozzle 19, which is fixed to the nozzle holder 44a by a hook.

The cool mist nozzle 19 is connected to a blast pump 77 by a blast tube 78. When the air sent from the blast pump 77 is sprayed from the blast tube 78, the heated water in the damper section 65 is drawn in by the Venturi effect thereby forming the cool mist C. The cool mist nozzle 19 sprays the cool mist C formed in such manner.

This will be described in detail. An air spraying portion 19c is arranged at the middle of the cool mist nozzle 19. The air spraying portion 19c sprays the flow of air conveyed through the blast tube 78 from the blast pump 77 toward the front. A water supply portion 19b is arranged spaced apart to the side from the distal end of the air spraying portion 19c. The water supply portion 19b has a distal end opening of which diameter is reduced to be sufficiently small. A negative pressure is generated by the flow of air sprayed from the air spraying portion 19c. The water is drawn out from the water supply portion 19b by the negative pressure, and such water forms the cool mist C. The cool mist C is sprayed toward the front by the flow of air sprayed from the air spraying portion 19c. In the present embodiment, the cool mist spray port 19a includes the air spraying portion 19c and the water supply portion 19b.

A cooling air inlet 72 is formed at the bottom surface of the main body 11 (housing 11a). A cooling motor 71, to which a cooling fan 70 is attached, is arranged at the lower side of the heat radiating member 68. The cooling fan 70 is rotatably driven by the cooling motor 71 and draws air through a dust filter 73 from the cooling air inlet 72. The dust filter 73 is set with a mesh roughness or an aperture ratio that does prevents the passage of dirt and dust. The cooling fan 70, the cooling motor 71, and the heat radiating member 68 (cooling unit 62) are accommodated in a cylindrical partition member 74. The cooling fan 70 sends a flow of air toward the heat radiating member 68, which is arranged on the downstream side, and cools the heat radiating member 68. The lower end opening of an accordion member 75 made of elastic material (e.g., silicone rubber) is attached to the upper part of the partition member 74. An upper end opening of the accordion member 75 is connected to a blast port 76. The blast port 76, which is concentric with the air spraying portion 19c of the cool mist nozzle 19, surrounds the cool mist nozzle 19. The partition member 74 and the accordion member 75 form a blast passage S1 for sending the flow of air sent from the cooling fan 70 to the blast port 76.

A drainage mechanism 80 will now be described.

As shown in Figs. 10 to 12, the drainage mechanism 80 is arranged at the lower part of the rear surface of the main body 11. The drainage mechanism 80 discharges the water that fills the tank 22, the tank holder 22a, the warm mist generation mechanism 40, and the cool mist generation mechanism 60 to the outside of the main body 11.

As shown in Fig. 10, a drainage cover 82 arranged in the housing 11a includes the drain outlet 24 and a drainage button 81 slidably operated in the vertical direction when the user P performs the drainage operation. The drain outlet cover 25 covers the drainage button 81 and the drain outlet 24 when the drain outlet cover 25 is closed.

As shown in Figs. 10 and 11, a finger hooking portion 81a of the drainage button 81 projects to the outer side of the housing 11a. The user P hooks a finger to the finger hooking portion 81a to operate the drainage button 81. Two hooks 81b are formed on the rear surface of the drainage button 81. The two hooks 81b engages an opening 82a formed in the drainage cover 82. The drainage button 81 is attached to the drainage cover 82 to be slidable in the vertical direction by the two hooks 81b. A quadratic prism-shaped projection 81c is formed between the hooks 81b.

A projection 82d facing upward is formed at the upper end of the drainage cover 82. The drainage cover 82 is fixed to the housing 11a by hooking the projection 82d to the housing 11a and fixing the lower end portion of the drainage cover 82 with a screw (not shown). Two parallel plate-shaped guide plates 82b, which extend in the vertical direction, are formed on the rear surface of the drainage cover 82. Two engagement portions 82c project from between the pair of guide plates 82b.

The guide member 83 is arranged between the guide plates 82b at the rear surface of the drainage cover 82. An engagement hole 83b, into which the projection 81c of the drainage button 81 is press-fitted, is formed at substantially the middle of the guide member 83. The drainage button 81 is attached to the drainage cover 82 by engaging the hook 81b and the engagement portion 82c. Thus, when the user P slides and operates the drainage button 81, the drainage button 81 and the guide member 83 integrally move in the vertical direction while being guided by the guide plates 82b of the drainage cover 82.

A drainage arm 84 is bent into a substantially L-shape. A basal end portion of the drainage arm 84 is fastened to the lower part of the guide member 83 by a screw N3. An engagement portion 84a having a notch is formed at the distal end portion of the drainage arm 84. The drainage arm 84 is movable in the vertical direction integrally with the guide member 83. In other words, when the user P slides and moves the drainage button 81 in the vertical direction, the drainage button 81, the guide member 83, and the drainage arm 84 integrally move upward and downward.

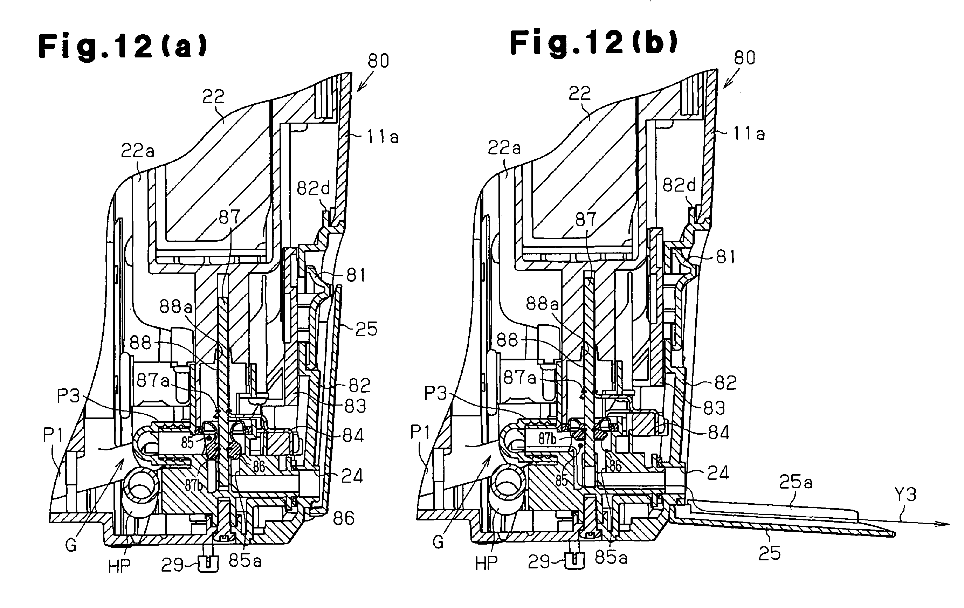

As shown in Fig. 12, a drainage chamber 85 is formed at the rear in the drain outlet 24. The drainage chamber 85 is connected to a drainage pipe P3 connected to the branched portion G. As shown with an arrow Y3 in Fig. 12, the water of the drainage pipe P3 passes through the drainage chamber 85 and through an opening 85a formed at the bottom of the drainage chamber 85 to be discharged from the drain outlet 24. The drainage pipe P3 and the drainage chamber 85 are arranged at positions lower (lower side) than the tank 22, the tank holder 22a, the warm mist generation mechanism 40, the cool mist generation mechanism 60, and the like.

The opening 85a of the drainage chamber 85 can be closed or opened by a drainage packing 86 serving as a seal member. The drainage packing 86 is switched between an open state (Fig. 12(b)) for discharging water and a closed state (Fig. 12(a)) for stopping the discharge of water in cooperation with the slide operation of the drainage button 81. This will be described in detail. A slide bar 87 is arranged at the central portion of the drainage chamber 85. The slide bar 87 is slidable along the longitudinal axis. A lower snap ring 87b is attached to the lower part of the slide bar 87. The inner side portion of the drainage packing 86 is attached to the lower snap ring 87b. When the slide bar 87 moves down, the inner side portion of the drainage packing 86 fixed to the lower snap ring 87b moves down and closes the opening 85a. When the slide bar 87 moves upward, the inner side portion of the drainage packing 86 fixed to the lower snap ring 87b moves upward and opens the opening 85a. The outer portion of the drainage packing 86 comes into close contact with the inner wall surface at the upper part of the drainage chamber 85 and prevents water from flowing out of the drainage chamber 85 and into the main body 11.

An upper snap ring 87a is attached at substantially the middle of the longitudinal axis of the slide bar 87. A spring receptacle 88a is formed at the lower part of the tank holder 22a, which is arranged on the upper side of the slide bar 87. The slide bar 87 is inserted into a coil spring 88. The coil spring 88 is arranged between the upper snap ring 87a and the spring receptacle 88a, and biases the slide bar 87 downward. That is, the spring receptacle 88a functions as an upper stopper of the coil spring 88, and the upper snap ring 87a functions as a lower stopper. The outer diameter of the upper snap ring 87a is set to be greater than the outer diameter of the coil spring 88.

The lower side of the upper snap ring 87a engages the engagement portion 84a of the drainage arm 84 of Fig. 11. Thus, when the drainage button 81 is not operated (Fig. 12(a)), the slide bar 87, the drainage arm 84, the guide member 83, and the drainage button 81 are held at the lower position by the downward biasing force of the coil spring 88. In this state, the drainage packing 86 closes the opening 85a. The downward biasing force of the coil spring 88 causes the inner portion of the drainage packing 86 to come into close contact with the edge of the opening 85a so as to maintain the closed state. When the user P upwardly moves the drainage button 81 (Fig. 12(b)), the drainage arm 84 also moves upward in cooperation, and the slide bar 87 hooked by the upper snap ring 87a also moves upward. This cancels the closed state (seal) of the opening 85a and discharges water.

The drainage arm 84 does not contact the drainage packing 86 even if the slide bar 87 moves to the lowermost position.

The fragrance generation unit 15 will now be described.

As shown in Fig. 13, the fragrance generation unit 15 includes the fragrance container HY, a volatilizing device 90, and a fragrance container removable mechanism 91. The fragrance container HY contains an aromatic substance. The volatilizing device 90 volatilizes the aromatic substance in the fragrance container HY. As shown in Fig. 13, a fragrance housing 93 forming an accommodation chamber 93a, which is open at substantially the middle of the top surface 12 and which can accommodate the fragrance container HY, is fixed to the housing 11a. A guide rib 93d is formed on the inner wall surface of the accommodation chamber 93a so as to extend in the vertical direction. A removal button sensor 93f of a lead switch type is arranged at the upper end of the guide rib 93d. The removal button sensor 93f is turned on by a magnet 123g arranged on the fragrance container HY when detecting that the fragrance container HY is accommodated in the accommodation chamber 93a, that is, the fragrance container HY is loaded in the fragrance generation unit 15.

A motor chamber 93b is defined at the lower side of the fragrance housing 93. A fragrance motor 95 is accommodated in the motor chamber 93b. The fragrance motor 95 is fastened to the fragrance housing 93 by a screw from the lower side with a circular motor holder 96. A drive shaft 95a of the fragrance motor 95 projects upward from the bottom surface of the accommodation chamber 93a. A fragrance fan 94 is fixed to the distal end of the drive shaft 95a by a bushing 95b. When the fragrance motor 95 rotates the fragrance fan 94, a flow of air is directed to the upper side of the accommodation chamber 93a. A packing 95c is arranged between the fragrance housing 93 and the fragrance motor 95. The packing 95c seals the accommodation chamber 93a and the motor chamber 93b so that they are not in communication with each other. In the present embodiment, the fragrance motor 95 and the fragrance fan 94 function as a volatilizing means.

A connection boss 93c is formed on the side surface at the lower part of the fragrance housing 93. The connection boss 93c is pipe-shaped and connected to the downstream of the fragrance fan 94. The connection boss 93c is connected to a pipe-shaped connection boss 98 by an air intake tube 97 made from an elastic material (e.g., silicone rubber). The connection boss 98 is in communication with an air intake port (suction portion) 99 formed at the bottom surface of the housing 11a. The inner diameter of the connection boss 98 substantially matches the diameter of the air intake port 99. A fragrance filter 100 that covers the air intake port 99 is arranged on the outer side at the bottom of the housing 11a. The mesh roughness or the aperture ratio of the fragrance filter 100 is set so as to prevent the passage of dirt and dust.

The accommodation chamber 93a, the connection boss 93c, the air intake tube 97, and the connection boss 98 form a blast passage S2 communicating the bottom surface of the housing 11a and the top surface 12. The fragrance fan 94 draws in air from the bottom surface of the main body 11 through the fragrance filter 100 when rotated by the fragrance motor 95 and sends the air toward the upper side of the accommodation chamber 93a through the blast passage S2.

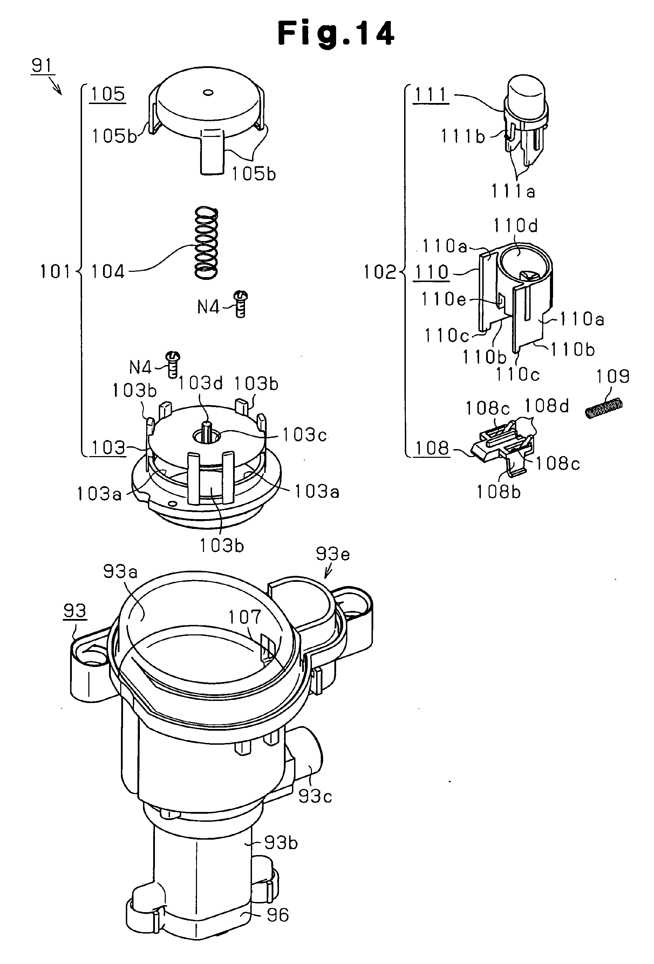

The fragrance container removable mechanism 91 will now be described. The fragrance container removable mechanism 91 removably attaches the fragrance container HY to the accommodation chamber 93a of the fragrance housing 93. As shown in Figs. 13 and 14, the fragrance container removable mechanism 91 includes a fragrance container lift section 101 for pushing the fragrance container HY upward, and a fragrance container fixing section 102 that engages or disengages the fragrance container HY and the accommodation chamber 93a.

The fragrance container lift section 101 will now be described.

As shown in Figs. 13 and 14, a fan cover 103 for covering the fragrance fan 94 is fastened to the accommodation chamber 93a by a screw N4. A plurality of (three in the present embodiment) guides 103b is formed in the fan cover 103 so as to extend in the vertical direction. A plurality of (three in the present embodiment) vent holes 103a through which the flow of air sent from the fragrance fan 94 passes is formed between the guides 103b of the fan cover 103. A circular recess, or spring receptacle 103c, and a boss 103d projecting upward from the middle of the spring receptacle 103c are formed at the upper part of the fan cover 103. A lift spring 104 is inserted into the boss 103d and accommodated in the spring receptacle 103c.

A cylindrical spring cover 105 is arranged on the upper side of the fan cover 103. The spring cover 105 is movable in the vertical direction with respect to the fan cover 103. The lift spring 104 biases the spring cover 105 upward.

The spring cover 105 has a top plate that covers the fan cover 103 and the lift spring 104. A tubular hollow boss 105a for receiving the boss 103d of the fan cover 103 is formed at the lower surface of the spring cover 105 (Fig. 13). The hollow boss 105a and the boss 103d are arranged in the lift spring 104 to guide the extension or contraction of the lift spring 104. The spring cover 105 has hooks 105b, the quantity of which is the same as the guides 103b of the fan cover 103. Each hook 105b extends downward from the side surface of the spring cover 105 and engages the corresponding guide 103b. The hook 105b restricts rotation of the spring cover 105 relative to the fan cover 103 and prevents the spring cover 105 from becoming loose in the upward direction.

The fragrance container lift section 101 includes the fan cover 103, the lift spring 104, and the spring cover 105.

The fragrance container fixing section (restriction means) 102 will now be described.

A cup-shaped button attachment portion 93e projects from the side surface at the upper part of the fragrance housing 93. The accommodation chamber 93a of the button attachment portion 93e is in communication with a hook projection hole 107. A plate-shaped hook guide lane 106 extending toward the hook projection hole 107 is arranged at the bottom of the button attachment portion 93e. A movable hook member 108 is arranged above the hook guide lane 106. As shown in Fig. 13(a), the fragrance container HY cannot be removed from the accommodation chamber 93a when the distal end of the hook member 108 engages with the fragrance container HY by the accommodation chamber 93a. A projection 108a projecting away from the accommodation chamber 93a is formed at the basal end of the hook member 108. The projection 108a is coupled to the inner wall of the button attachment portion 93e by a removable spring 109. The removable spring 109 biases the hook member 108 toward the accommodation chamber 93a. A downwardly projecting piece 108b extends from each of the two side surfaces of the hook member 108. The lower end of each projecting piece 108b is bent outward and extended horizontally. The hook member 108 has two parallel elongated holes 108c extending in the projecting direction of the hook member 108. The two elongated holes 108c each include an inclined portion 108d so that the two inclined portions 108d sandwich the projection 108a. Each inclined portion 108d has an inclined plane inclined downward from the rear toward the front of the hook member 108.

A cylindrical button holder 110 arranged on the upper side of the hook member 108 fixes the hook member 108 from above. The button holder 110 is press-fitted to the button attachment portion 93e and fixed at a position lower than the top surface 12. The side surface of the button holder 110 includes two wall portions 110a extending toward the lower side. The two wall portions 110a face each other. A lower end face 110b of each wall portion 110a is arranged parallel to the upper surface of the projecting piece 108b to restrict movement of the hook member 108 in the vertical direction. A projection stopping wall 110c, which downwardly projects from each lower end face 110b, contacts the projecting piece 108b of the hook member 108 and is biased by the removable spring 109. The contact of the projecting piece 108b and the projection stopping wall 110c prevents the hook member 108 from falling out of the hook projection hole 107 and into the accommodation chamber 93a with the biasing force of the removable spring 109. A fragrance container discharge button 111 pressed by the user P is attached to a button accommodating portion 110d formed on the inner side of the button holder 110. Two rectangular cutouts 110e are formed at positions facing each other at the lower part of the button holder 110.

The fragrance container discharge button 111 has two downwardly extending ribs 111a. The two ribs 111a are respectively engaged with the inclined portions 108d of the hook member 108. The lower end face of each rib 111a is an inclined plane corresponding to the inclined surface of the inclined portion 108d. The inclined surface of the rib 111a inclines to the lower side from the rear toward the front of the hook member 108. When the fragrance container discharge button 111 is pressed, the inclined plane of each rib 111a slides while contacting the inclined portion 108d of the hook member 108 to move the hook member 108 toward the rear side (away from the accommodation chamber 93a). The distal end portion of the hook member 108 does not project out of the accommodation chamber 93a. The biasing force of the removable spring 109 that biases the hook member 108 toward the accommodation chamber 93a biases the fragrance container discharge button 111 upward. A hook 111b is formed between the ribs 111a of the fragrance container discharge button 111. The hook 111b is engaged with the cutout 110e of the button holder 110. Thus, the fragrance container discharge button 111 is vertically movable and does not become loose in the upward direction.

Therefore, the fragrance container fixing section 102 includes the hook member 108, the removable spring 109, the button holder 110, and the fragrance container discharge button 111.

The fragrance container HY for containing an aromatic substance will now be described.

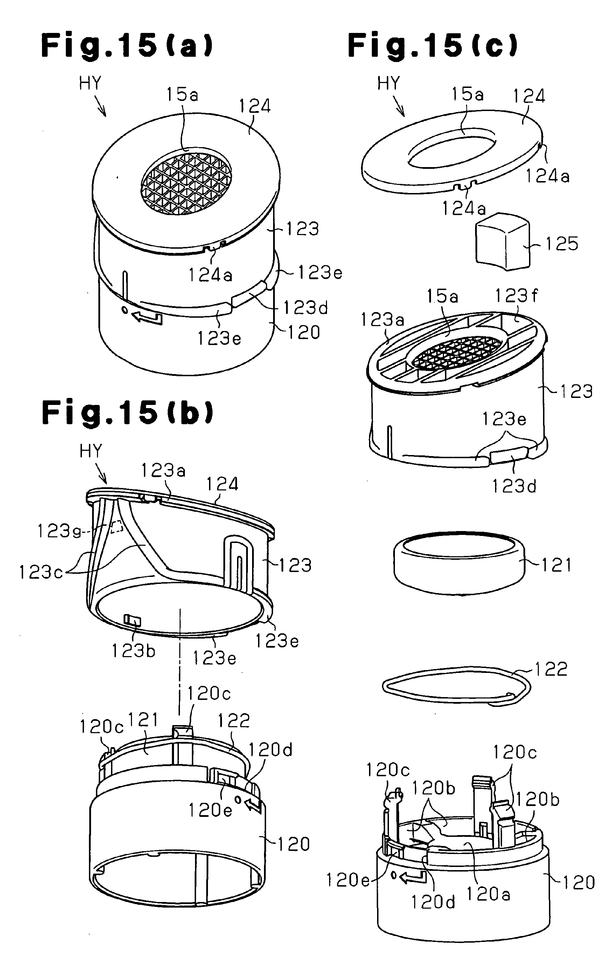

As shown in Figs. 13 and 15, the fragrance container HY mainly includes a holder 120 set on the spring cover 105 arranged in the accommodation chamber 93a, a fragrance plate (bottomed container) 121 for accumulating the aromatic substance, and a lid member 123 attached to the holder 120.

The holder 120 has a tubular shape. A support 120a for supporting the bottom of the fragrance plate 121 from underneath and a ventilation path 120b formed at the periphery of the support 120a are formed at the upper part of the holder 120. The ventilation path 120b communicates the spring cover 105 side and the fragrance plate 121 side. The support 120a is supported by the contact of the lower surface of the support 120a and the spring cover 105 in the accommodation chamber 93a. The holder 120 holds the fragrance plate 121 and retains the fragrance plate 121 in a predetermined position and orientation. The holder 120 includes a plurality of (three in the present embodiment) upwardly projecting hooks 120c. The hooks 120c are formed at every predetermined interval (about 120 degrees in the present embodiment) in the circumferential direction of the holder 120. The fragrance plate 121 is detachably fitted to the hook 120c. In the present embodiment, the fragrance plate 121 and the holder 120 function as a bottomed container member. A ring 122 made of metal is attached to the hook 120c with the fragrance plate 121 fitted thereto. Two cutouts 120d extending in the circumferential direction are formed at part of the upper end of the holder 120. Each cutout 120d has a recess 120e. The size of the holder 120 is determined so as to prevent accidental swallowing by infants in a state in which the fragrance plate 121 is attached.

A cylindrical lid member 123 is detachably attached to the holder 120. A top plate portion 123a of the cylindrical lid member 123 is inclined with respect to the horizontal plane. The inclination angle of the top plate portion 123a is the same as the inclination angle of the top surface 12. This will now be described in detail. Two projections 123b are formed on the inner surface at the lower part of the lid member 123. Each projection 123b of the lid member 123 is inserted into the corresponding cutout 120d of the holder 120 from above, and the lid member 123 is rotated in the circumferential direction so that the two projections 123b are fitted to the two recesses 120e of the holder 120, respectively. The engagement state is released by rotating the lid member 123 in the opposite direction from the state in which the projection 123b and the recesses 120e are fitted together. When the lid member 123 is attached to the holder 120, the lid member 123 covers the hook 120c, the fragrance plate 121, and the ring 122. A fragrance emission port 15a for emitting the volatilized fragrance is formed at the middle of the top plate portion 123a of the lid member 123. In the illustrated example, the fragrance emission port 15a is formed to have a grid-form. The size of the mesh of the grid formed in the fragrance emission port 15a is set to a size the flow of air conveyed by the fragrance fan 94 is not inhibited and a size the liquid aromatic substance is held at the mesh of the grid and is less likely to pass therethrough due to surface tension when attached to the grid structure. The size of the mesh of the grid of the fragrance emission port 15a is set to a size (fineness) that suppresses the user P from dropping aromatic substance to the fragrance plate 121 from above the fragrance emission port 15a. In the present embodiment, the size (length of one side) of the mesh of the grid is set to between 0.5 and 4.0 mm, and preferably between 1.8 and 2.2 mm.

Rib-shaped guide lanes 123c are formed at the side surface of the lid member 123. The two adjacent guide lanes 123c become more spaced apart at lower positions. When inserting the fragrance container HY into the accommodation chamber 93a, the guide lane 123c and the guide rib 93d formed at the accommodation chamber 93a guide the fragrance container HT until reaching the position where the inclination of the top plate portion 123a and the inclination of the top surface 12 (main body 11) match.

The magnet 123g is arranged between the two adjacent guide lanes 123c. The removal button sensor 93f detects magnetic flux of the magnet 123g when the fragrance container HY is loaded in the accommodation chamber 93a. A collar 123e at the lower end of the lid member 123 extends in the peripheral direction and forms a ventilation path. A rubber installing portion 123f partitioned by a plurality of rib structures is formed at the upper part of the lid member 123. A rubber member 125 formed to a substantially rectangular solid shape is inserted into the rubber installing portion 123f. The lower end portion of the rubber member 125 projects out from a peep hole (not shown) formed in the lid member 123, and elastically contacts the inner wall surface of the accommodation chamber 93a and depresses as if being pushed toward the lid member 123 side with the fragrance container HY inserted to the accommodation chamber 93a.

As shown in Fig. 13, a direction changing unit 126 at the inner side of the lid member 123 changes the flow of air (arrow Y4) blown by the fragrance fan 94 to the direction of flowing into the interior of the fragrance plate 121 from the periphery of the fragrance plate 121. This will now be discussed in detail. The direction changing unit 126 is formed at the inner side of the top plate portion 123a of the lid member 123.

When the fragrance container HY is inserted into the accommodation chamber 93a, the distal end of the hook member 108, which is described above, is snap-fitted with a protrusion 123d projecting outward from the side surface of the lid member 123, and fixes the fragrance container HY. If the fragrance container discharge button 111 is pressed in such a state, the hook member 108 moves and releases the snap-fit with the protrusion 123d. The fragrance container HY is pushed upward by the biasing force of the spring cover 105 and projects from the top surface 12 to a height enabling the user P to hold the fragrance container HY.

A container cover 124 is attached to the upper part of the lid member 123. The container cover 124 has an annular shape with a fragrance emission hole at the middle. A plurality of (four in the present embodiment) bent portions 124a is formed at the outer edge of the container cover 124. The distal end of each bent portion 124a is bent inward and engages the lid member 123. The container cover 124 is fixed to the lid member 123 by the bent portions 124a.

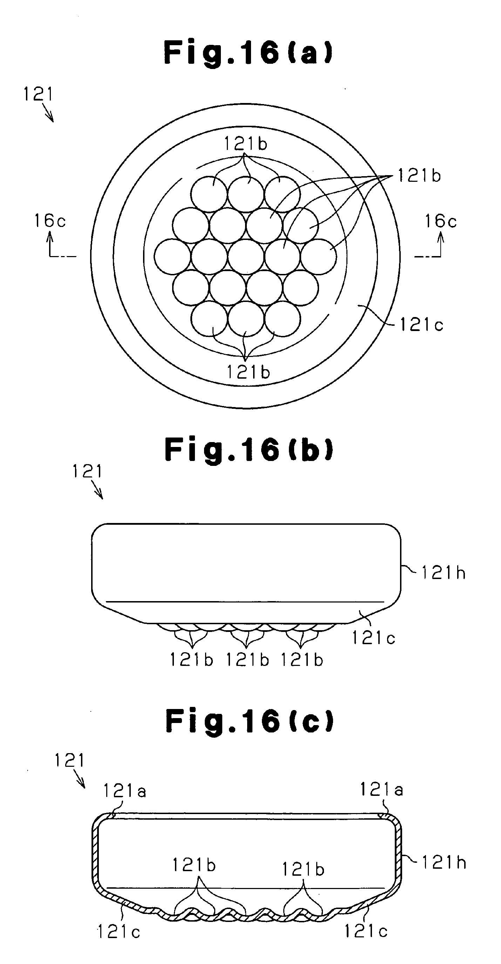

The fragrance plate 121 for holding the aromatic substance will now be discussed with reference to Fig. 16.

As shown in Fig. 16, the fragrance plate 121 made of metal (e.g., stainless material) has a cylindrical shape and includes a circular bottom and a side wall 121h. The opening edge of the fragrance plate 121 is partitioned by a fold-back portion 121a folded back toward the inner side. The length from the side wall 121h to the edge of the fold-back portion 121a is defined as the length of the fold-back portion 121a. The length of the fold-back portion 121a is determined such that two drops, and preferably, ten or more drops of aromatic substance in the fragrance plate 121 do not spill out when the fragrance plate 121 is vertically standing. In the present embodiment, the outer diameter of the fragrance plate 121 has a diameter of 28 mm, and the opening diameter of the fold-back portion 121a is 24 mm. The height of the fragrance plate 121 is 10 mm.

A plurality of (19 in the present embodiment) recesses 121b (accumulating portions), which are semispherical, are formed at the bottom of the fragrance plate 121. An inclined portion 121c is formed between the side wall 121h and the recess 121b. The inclined portion 121c inclines to become higher toward the outer side. The recess 121b and the inclined portion 121c adjust the contact area (hereinafter referred to as "surface area of aromatic substance") of the aromatic substance in the fragrance plate 121 and the flow of air. Generally, there is a correlation between the surface area of the aromatic substance and the volatilization volume. The volatilization volume of the aromatic substance becomes greater as the surface area of the aromatic substance becomes greater, and the volatilization volume becomes less as the surface area of the aromatic substance becomes less. The recess 121b and the inclined portion 121c of the present embodiment function as an adjustment means for adjusting the volatilization volume of the aromatic substance by adjusting the surface area of the aromatic substance accumulated in the fragrance plate 121. This will now be described in detail. The aromatic substance dropped into the fragrance plate 121 flows into each recess 121b and does not spread to the bottom excluding the recess 121b. That is, if the amount of the aromatic substance does not exceed the sum of the volume of all of the recesses 121b (hereinafter referred to as "recess volume"), the recess 121b adjust the surface area of the aromatic substance in correspondence with the area of all of the recesses 121b. If the aromatic substance of an amount exceeding the recess volume is dropped into the recess 121b, the aromatic substance that cannot be held in the recess 121b spreads to the bottom excluding the recess 121b but is adjusted by the inclined portion 121c so that the surface area is in correspondence with the amount of aromatic substance.

The size (diameter and depth) of the recess 121b and the inclined portion 121c are set such that the ratio of the surface areas is a predetermined ratio for when the dropped amount of the aromatic substance is for a single drop and when the dropped amount is for two drops, that is, such that there is a constant correlation between the number of droplets of the aromatic substance and the surface area. For example, the ratio of the surface area of when the dropped amount of the aromatic substance is one drop and the surface area of when the dropped amount is two drops is 1:1.3 to 3.0, and preferably 1:1.5 to 2.0. In the present embodiment, substantially all the recesses 121b are filled with aromatic substance if one drop of aromatic substance is dropped into the recess 121b. In the present embodiment, the aromatic substance spreads to the lower end (boundary portion) of the inclined portion 121c if two drops of aromatic substance are dropped into the recess 121b. In the present embodiment, the surface area enlarges along the inclination of the inclined portion 121c if three drops of aromatic substance are dropped into the recess 121b.

The aromatic substance accumulated in the recess 121b remains in the recess 121b even if the fragrance plate 121 is tilted, and is less likely to flow out from the recess 121b.

In the fragrance generation unit 15, the combination conditions of the diameter of the fragrance emission port 15a, the flow rate of air sprayed from the fragrance emission port 15a, and the surface area of the aromatic substance in the fragrance plate 121 is set such that the concentration of the aromatic substance mixed in each of the mists H and C and inhaled by the user P does not exceed a predetermined concentration (e.g., 10 ppm), and such that the flow (spray) of mists H and C is not inhibited. As for the combination condition, in the present embodiment, the fragrance fan 94 is rotatably driven such that the opening diameter of the fragrance emission port 15a is 19 mm, and the flow rate of air at the fragrance emission port 15a is less than or equal to 0.6 m/sec and more preferably less than or equal to 0.3 m/sec. The recess 121b and the inclined portion 121c of the fragrance plate 121 are configured such that the surface area of the aromatic substance is between 100 and 220 mm², and more preferably, between 130 and 190 mm² when the dropped amount of aromatic substance is a single drop (about 0.03 ml). The recess 121b and the inclined portion 121c of the fragrance plate 121 are configured such that the surface area of the aromatic substance is between 220 and 340 mm², and more preferably, between 250 and 310 mm² when the dropped amount of aromatic substance is two drops.

A control circuit of the cosmetic device 10 will now be described.

As shown in Fig. 3, a control circuit board 31 is arranged below the operation unit 14 in the main body 11. The control circuit board 31 controls the entire operation of the cosmetic device 10. A power supply circuit board 30 is fixed below the control circuit board 31. The power supply circuit board 30 supplies power to the control circuit board 31, various motors, various heaters, and the like.

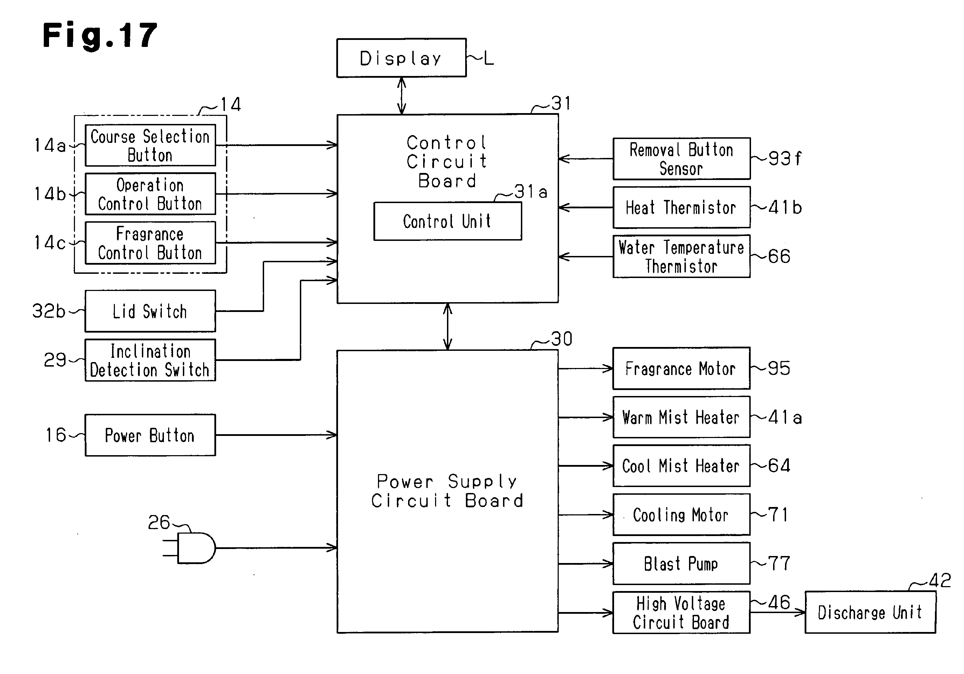

An electric circuit of the cosmetic device 10 will now be described with reference to Fig. 17.

### <Power supply circuit board 30>

The power supply circuit board 30 is connected to a power supply cord 26 through which power is supplied from a commercially available outlet. The power supply circuit board 30 is connected to the power button 16. Whenever the power button 16 is pressed, the power supply circuit board 30 is switches between an off state, in which it stops the supply of power from the commercially available outlet, and an on state, in which is supplies operational power to the cosmetic device 10.

The power supply circuit board 30 is connected to the fragrance motor 95, the warm mist heater (sometimes simply referred to as heater) 41a, the cool mist heater 64, the cooling motor 71, and the blast pump 77. The power supply circuit board 30 is connected to the discharge unit 42 by the high voltage circuit board 46. The high voltage circuit board 46 increases the voltage of the power supplied from the power supply circuit board 30 to a predetermined voltage that can be discharged from the discharge needle 48 and supplies the voltage to the discharge unit 42. The power supply circuit board 30 is connected to the control circuit board 31. The power supply circuit board 30 supplies power based on the control of the control circuit board 31 to drive and stop various motors, various heaters, and the like.

### <Control circuit board 31>

The control circuit board 31 includes a control unit 31a for storing programs for controlling the operation of the cosmetic device 10 and executing various calculation processes. The control unit 31a is a microcomputer or the like. The control circuit board 31 is connected to the operation control button 14b, the operation mode selection button 14a, and the fragrance control button 14c to receive operation signals from the buttons 14a to 14c.

The control circuit board 31 is connected to the main body lid switch 32b. The main body lid switch 32b detects a state in which the main body lid 13 opens and provides an on signal to the control circuit board 31. The control unit 31a validates the pressing-operation of the operation control button 14b, the operation mode selection button 14a, and the fragrance control button 14c in response to the on signal from the main body lid switch 32b. If the on signal is not input from the main body lid switch 32b, that is, if the main body lid 13 is in the closed state, the control unit 31a invalidates the pressing-operation of the buttons 14a to 14c of the operation unit 14. The main body lid switch 32b thus functions as a safety switch.

The control circuit board 31 is connected to the removal button sensor 93f. The removal button sensor 93f detects when the fragrance container HY is accommodated (fixed) in the accommodation chamber 93a and provides an on signal to the control circuit board 31. The control unit 31a validates pressing-operation of the operation control button 14b, the operation mode selection button 14a, and the fragrance control button 14c in response to the on signal from the removal button sensor 93f. If the on signal is not input from the removal button sensor 93f, that is, if the fragrance container HY is not accommodated (fixed) in the accommodation chamber 93a, the control unit 31a invalidates the pressing-operation of the buttons 14a to 14c of the operation unit 14. The removal button sensor 93f thus functions as a safety switch.

The control unit 31a controls the power supply circuit board 30 to stop generation of the mists H and C when the on signal is not provided from the removal button sensor 93f in a state in which the mists H and C are being sprayed from the mist nozzles 18 and 19. This will now be described in detail. The control unit 31a controls the power supply circuit board 30 to turn off the warm mist heater 41a during the generation of the warm mist H. The control unit 31a also controls the power supply circuit board 30 to turn off the blast pump 77, the cool mist heater 64, and the cooling motor 71 during the generation of the cool mist C.

The control circuit board 31 is connected to the inclination detection switch 29. The inclination detection switch 29 provides the on signal to the control circuit board 31 when the main body 11 falls aside or when the main body 11 is lifted. The control unit 31a controls the power supply circuit board 30 to stop the supply of power to the warm mist heater 41a, the cool mist heater 64, the cooling motor 71, the blast pump 77, and the high voltage circuit board 46 in response to the on signal from the inclination detection switch 29. That is, the control unit 31a stops all operations of the cosmetic device 10 in response to the on signal from the inclination detection switch 29 to ensure the safety of the user.

The control circuit board 31 is connected to the display L. The control unit 31a controls the display L so as to light the light emitting body at a predetermined lighting pattern according to the control state of the cosmetic device 10.

The control circuit board 31 is connected to the heater thermistor 41b and the water temperature thermistor 66. Each of the thermistors 41b and 66 provides a temperature detection signal corresponding to the measured temperature to the control circuit board 31. The control unit 31a controls the power supply circuit board 30 to stop the supply of power to the warm mist heater 41a and turns off the warm mist heater 41a when the temperature of the warm mist heater 41a detected by the heater thermistor 41b exceeds a predetermined set temperature. The control unit 31a controls the power supply circuit board 30 to supply power to the cool mist heater 64 and turns on the cool mist heater 64 when the water temperature of the heating processing unit 61 detected by the water temperature thermistor 66 becomes lower than a predetermined sterilization temperature (80°C in the present embodiment). The control unit 31a controls the power supply circuit board 30 to stop the supply of power to the cool mist heater 64 and turns off the cool mist heater 64 when the water temperature of the heating processing unit 61 detected by the water temperature thermistor 66 becomes higher than or equal to the predetermined sterilization temperature.

The control circuit board 31 is connected to the power supply circuit board 30. The control circuit board 31 is supplied with power from the power supply circuit board 30. The control circuit board 31 controls the power supply circuit board 30 and controls the supply of power to various motors, various heaters, and the like connected to the power supply circuit board 30.

The control unit 31a controls the power supply circuit board 30 so that the rotation (speed) of the fragrance fan 94 corresponds to the fragrance concentration, which is selected by the user P by operating the fragrance control button 14c, and supplies the controlled power to the fragrance motor 95. The control unit 31a controls the power supply circuit board 30 such that the speed of the fragrance fan 94 is higher when the selected fragrance concentration is higher. In other words, the control unit 31a controls the power supply circuit board 30 such that the amount of air conveyed by the fragrance fan 94 increases when the selected fragrance concentration is higher. The control unit 31a increases or decreases the amount of aromatic substance that is volatilized at predetermined time intervals.

The control unit 31a of the present embodiment increases or decreases the amount of aromatic substance that is volatilized at predetermined time intervals. This will now be described in detail. The control unit 31a causes the power supply circuit board 30 to drive the fragrance motor 95 based on the intensity (concentration) of the fragrance selected by the user P. Furthermore, the control unit 31a controls the power supply circuit board 30 to stop the fragrance motor 95 for a predetermined period (five seconds in the present embodiment) when a predetermined period (20 seconds in the present embodiment) elapsed from when the fragrance motor 95 starts being driven. With such a configuration, the cosmetic device 10 alternately generates and stops generating the fragrance.

In the present embodiment, the control unit 31a for controlling the power supply circuit board 30 to drive various motors and various heaters serve as an adjustment means.

The operation of the cosmetic device 10 will be described in detail below.

### <Cap 21>

When the cosmetic device 10 is in a normal state of use, the cap 21 is arranged at the spray position, as shown in the state of Fig. 6(a). When the user P uses his or her hand PH to lower the cap 21, the cap 21 moves to the protective position, as shown in the state of Fig. 6(b). At the protective position, the cap 21 is arranged between the mist nozzles 18 and 19 and the hand PH. This prevents the mists H and C from directly striking the hand PH of the user P. Thus, the cap 21 in the protective position physically separates the hand PH of the user P from the mist nozzles 18 and 19.

In the protective position, the cap 21 covers the upper side of the mist nozzles 18 and 19. Thus, the spraying directions of the mists H and C sprayed from the mist nozzles 18 and 19 are lowered from the normal state of use. In this state, and the mists H and C are not sprayed toward the user P.

### <Warm mist generation mechanism 40>

As shown in Fig. 7, the water supplied from the tank 22 to the tank holder 22a is further supplied to the boiling chamber 41c through the water supply passage K1 and the water supply passage K2. The water supply from the tank 22 maintains the water level of the boiling chamber 41c at a predetermined water level W. This will now be described in detail. The water supply from the tank 22 starts when the water level of the boiling chamber 41c falls, and the water supply from the tank 22 stops when the water level of the boiling chamber 41c exceeds the predetermined height (water level W). The warm mist heater 41a heats and vaporizes the water supplied to the boiling chamber 41c to generate the warm mist. The generated warm mist is guided to the upper side through the warm mist path M1 and atomized (ionized) by the high voltage discharge carried out between the discharge needles 48 in the discharge unit 42. The atomized warm mist is sprayed from the warm mist nozzle 18 toward the front. The warm mist heater 41a is turned off by the control of the control unit 31a when the water level W of the boiling chamber 41c falls and the temperature rises such as when the tank 22 becomes empty.

### <Cool mist generation mechanism 60>

As shown in Fig. 9, the water supplied from the tank 22 to the tank holder 22a is supplied to the reservoir chamber 61a of the heating processing unit 61 through the water supply passage K3. The water supplied to the reservoir chamber 61a flows into the damper section 65 from the inlet port 65a and is maintained at the water level W. The cool mist heater 64 heats the water of the reservoir chamber 61a including the water that flows into the damper section 65 to a predetermined sterilization temperature (80°C in the present embodiment).

In the damper section 65, the partition plates 65c arranged alternately at predetermined intervals suppresses the convention of heated water. The inlet port 65a is formed to have the minimum area. This suppresses the flow of low-temperature water supplied from the water supply passage K3 into the damper section 65 due to convection. The water in the damper section 65 is thus less likely to be subjected to the influence of low-temperature water and may be stably heated by the cool mist heater 64.

The water that is to be sprayed from the cool mist nozzle 19 is accumulated in the damper section 65 for a time period that satisfies at least the sterilization condition (30 seconds in the present embodiment). The cosmetic device 10 of the present embodiment does not include an operation mode in which only the cool mist C is sprayed. Thus, the sterilization condition of the water of the damper section 65 is achieved when spraying the warm mist H from the warm mist nozzle 18. After ending the spraying of the warm mist, the cool mist nozzle 19 generates the cool mist from the sterilized water to readily spray the cool mist toward the user P.

When the blast pump 77 is turned on (driven) and air is sprayed from the air spraying portion 19c, the water, which is heated and sterilized in the damper section 65, is drawn toward the water supply portion 19b through the water supply passage K4 by the negative pressure generated during spraying. The water is then atomized into cool mist and sprayed toward the front.

The bubbles mixed in the water, which is drawn out of the damper section 65, are captured and exchanged with water in the gas-liquid exchange unit 67. The control unit 31a controls the power supply circuit board 30 such that the blast pump 77 is driven at a low speed for a predetermined period (five seconds in the present embodiment) from the when generation of the cool mist starts (the blast pump 77 goes on) with the cool mist nozzle 19. After a predetermined period elapses, the control unit 31a controls the power supply circuit board 30 such that the blast pump 77 is driven at a normal speed. In other words, the control unit 31a controls the operation of the blast pump 77 so that the water of the damper section 65 slowly flows into the gas-liquid exchange chamber 67a when generation of the cool mist starts. This first sprays all the air out of the gas-liquid exchange chamber 67a from the cool mist nozzle 19. The sudden flow of water into the gas-liquid exchange chamber 67a prevents the spraying of the cool mist C from being started when air still remains in the gas-liquid exchange chamber 67a. The water in the water supply passage K4 is prevented from being divided by the bubbles. Further, intermittent spraying and temporary suspension of the cool mist C are prevented.

The water from which bubbles are eliminated is cooled when passing through the water supply passage K4 of the heat radiating member 68. The heat radiating member 68 is cooled by the air conveyed by the cooling fan 70. The flow of air conveyed by the cooling fan 70 is sprayed toward the user P from the blast port 76, which opens at a side of the cool mist nozzle 19, through the blast passage S1. The spraying of air toward the user P from the blast port 76 enhances the vaporization of cool mist on the skin of the user P. This efficiently cools the skin and the user P will feel pleasant cooling of the skin.

### <Fragrance generation unit 15>

When the fragrance container discharge button 111 is pressed down in the state of Fig. 13, the hook member 108 moves away from the accommodation chamber 93a (fragrance container HY) and disengages the distal end of the hook member 108 from the protrusion 123d of the lid member 123. The spring cover 105, which receives the biasing force of the lift spring 104, lifts the fragrance container HY and projects it out of the top surface 12. The user P may remove the fragrance container HY from the fragrance generation unit 15 (accommodation chamber 93a) by lifting the projected fragrance container HY. In the illustrated embodiment, the fragrance container HY is removed in a removal direction that crosses the spraying direction of the mists H and C.

As shown in Figs. 15(a) and 15(b), when the lid member 123 is rotated in the circumferential direction, the projection 123b of the lid member 123 and the recess 120e of the holder 120 are disengaged. This detaches the lid member 123 from the holder 120. The user P may drop the desired aromatic substance into the recess 121b and the inclined portion 121c of the fragrance plate 121. In this case, the spreading of the aromatic substance dropped onto the bottom surface of the fragrance plate 121 may be adjusted by the recess 121b and the inclined portion 121c. In other words, the surface area of the aromatic substance corresponding to the number of drops of the aromatic substance is automatically obtained.

After dropping the aromatic substance onto the fragrance plate 121, the lid member 123 covering the fragrance plate 121 is set, and then rotated in the circumferential direction to engage the projection 123b and the recess 120e. This attaches the lid member 123 to the holder 120.

The fragrance container HY is inserted into the accommodation chamber 93a (main body 11) in a state in which the guide lane 123c of the lid member 123 and the guide rib 93d of the accommodation chamber 93a are aligned. In this case, the protrusion 123d of the lid member 123 pushes the distal end of the hook member 108 so as to move away from the fragrance container HY (accommodation chamber 93a) when the upper surface (container cover 124) of the fragrance container HY is pushed downward. Furthermore, the hook member 108 biased by the removable spring 109 projects out to the accommodation chamber 93a and engages with the protrusion 123d when the fragrance container HY is pushed and moved downward. The fragrance container HY is then fixed (attached) to the accommodation chamber 93a (main body 11).

As shown in Fig. 13, when power is supplied from the power supply circuit board 30 to the fragrance motor 95, the fragrance fan 94 is rotated and air is drawn from the air intake port 99 into the blast passage S2 (arrow Y4). The air drawn into the blast passage S2 is further conveyed upward by the fragrance fan 94 (arrow Y4). The flow of air generated by the fragrance fan 94 is conveyed upward through the side of the spring cover 105 from the vent hole 103a of the fan cover 103. Then, the direction changing unit 126 formed on the inner surface of the lid member 123 changes the direction of the air so that it flows into the fragrance plate 121. The air flowing into the fragrance plate 121 volatilizes the aromatic substance in the recess 121b and the inclined portion 121c of the fragrance plate 121. Then, the air flows upward and is emitted from the fragrance emission port 15a (arrow Y4). The flow rate, or velocity, of the air sprayed upward from the fragrance emission port 15a is set so as to not to interfere with the mist sprayed from each of the mist nozzles 18 and 19. The maximum velocity is 0.6 m/sec in the present embodiment.

As shown in Figs. 18(a) and 18(b), the warm mist is sprayed toward the face of the user P from the warm mist nozzle 18 (warm mist spray port 18a) when the cosmetic device 10 is in a state of use. The cool mist C is sprayed toward the face of the user P from the cool mist nozzle 19 (cool mist spray port 19a). Each of the mists H and C are diffused in the horizontal direction and the vertical direction as it moves forward. Thus, each of the mists H and C are diffused in a wider range at positions farther from the mist nozzles 18 and 19.

The fragrance generated in the fragrance generation unit 15 is emitted (emitting direction H1 of Fig. 18) upward from the fragrance emission port 15a and mixed with the mists H and C passing the upper side of the fragrance emission port 15a. The fragrance is then conveyed toward the user P together with the mists H and C (arrow A of Fig. 18). Therefore, the spraying direction H2 of the mist emitted from the mist nozzles 18 and 19 is set to interest the emitting direction H1 of the fragrance emitted from the fragrance emission port 15a.

The procedures for using the cosmetic device 10 of the present embodiment will now be described.

The user P places the cosmetic device 10 on a horizontal desk or the like, opens the main body lid 13, and plugs the power supply cord 26 into a commercially available outlet (not shown) to supply power to the cosmetic device 10. The user P presses the power button 16 so that the cosmetic device 10 is in standby. The user P then presses the operation mode selection button 14a to select the desired operation mode.

The user P presses the fragrance control button 14c to select the intensity (concentration) of the desired fragrance. In response to the user P pressing the operation control button 14b after selecting the operation mode and the intensity of the fragrance, the cosmetic device 10 sprays the mists H and C and emits (generates) the fragrance in accordance with the operation mode and fragrance intensity, which are selected by the user P.

This will now be described in detail. The warm mist H is first sprayed toward the user P from the warm mist nozzle 18 to warm the skin of the user P. After a predetermined period elapses from when the spraying of the warm mist H starts, the spraying of the warm mist is stopped. At about the same time, the cool mist C is sprayed toward the user P from the cool mist nozzle 19 to cool the skin of the user P. After a predetermined period elapses from when the spraying of the cool mist C starts, the spraying of the cool mist C is stopped. At about the same time, the spraying of the warm mist H is started. The spraying of the mists is terminated after the spraying of the warm mist H and the spraying of the cool mist C are alternately repeated for a predetermined number of times. During the spraying of the mists H and C, fragrance is emitted (generated) from the fragrance emission port 15a with the selected fragrance intensity.

The user P may switch the intensity (concentration) of the fragrance even when the mists H and C are being sprayed by pressing the fragrance control button 14c. The user P may also stop the spraying of the mists from the mist nozzles 18 and 19 even when the mists H and C are being sprayed by pressing the power button 16 or the operation control button 14b.

The operation of the cosmetic device 10 is disabled by closing the main body lid 13 after using the cosmetic device 10.

Therefore, the present embodiment has the advantages described below.
(1) Fragrance is emitted toward each of the mists H and C from locations separated from the mist nozzles 18 and 19. Thus, the mists H and C sprayed from the mist nozzles 18 and 19 are efficiently mixed and blended with the fragrance. This stably sends (feeds) the fragrance in the spraying direction of the mists H and C. Therefore, when the mists H and C are sprayed from the mist nozzles 18 and 19 onto the face or the like, the user P of the cosmetic device 10 is provided with a stable fragrance.
(2) The fragrance emission port 15a is located closer to the user P than the mist nozzles 18 and 19 in the vicinity of the mist nozzles 18 and 19. Thus, the fragrance is emitted toward the mists H and C before the mists H and C are diffused when sprayed toward the user P. Therefore, the fragrance and the mists H and C easily mix compared to when the fragrance emission port 15a is spaced apart from the mist nozzles 18 and 19 and when the fragrance is emitted toward the mists H and C that have already been diffused. The user P using the cosmetic device 10 then obtains stable fragrance when the mists H and C are sprayed from the mist nozzles 18 and 19 to the face or the like.
(3) The fragrance emission port 15a is arranged closer to the user P than the mist nozzles 18 and 19 and located between the middle of the warm mist spray port 18a (warm mist nozzle 18) and the cool mist spray port 19a (cool mist nozzle 19). Thus, the fragrance is emitted from the fragrance emission port 15a toward both of the warm mist H and the cool mist C. This mixes and blends the mists H and C sprayed from the mist nozzles 18 and 19 with the fragrance. Further, the intensity (concentration) of the fragrance remains uniform in the mists H and C. Further, the user P obtains a stable fragrance regardless which one of the mist nozzle 18 and 19 is used.
(4) A plurality of recesses 121b and an inclined portion 121c are formed at the bottom of the fragrance plate 121. Thus, when the aromatic substance of an amount that does not exceed the volume of the recess is dropped into the recess 121b, the aromatic substance flows into the plurality of recesses 121b, and the surface area is adjusted so as not to be wider than the portion including the recesses 121b. When the aromatic substance of an amount exceeding the volume of the recess is dropped into the recess 121b, the surface area of the aromatic substance is adjusted in correspondence with the dropped amount of aromatic substance along the inclination of the inclined portion 121c. Therefore, the surface area of the aromatic substance dropped (accumulated) in the fragrance plate 121 is adjusted in correspondence with the dropped amount of aromatic substance to adjust the volatilization volume of the aromatic substance.
(5) The control unit 31a controls the power supply circuit board 30 to drive the fragrance motor 95 at a predetermined speed in accordance with the intensity (concentration) of the fragrance set by the user P. The volatilization volume of the aromatic substance is adjusted to obtain the desired intensity (concentration) of the fragrance. Furthermore, since the surface area of the aromatic substance in the fragrance plate 121 is within a predetermined range in correspondence with the number of drops of the aromatic substance, finer adjustment for the intensity (concentration) of the fragrance is realized.
(6) Generation of the fragrance is alternately commenced and suspended. Generally, the olfactory intensity for fragrance decreases when a human continuously smells the fragrance of a constant concentration. Thus, it becomes difficult for a human to sense the fragrance. With the cosmetic device 10 of the present embodiment, the olfactory intensity of the user P on the aromatic substance is periodically recovered thereby enabling the user P to continuously sense the fragrance.
(7) The aromatic substance in the fragrance plate 121 is volatilized by the blast from the fragrance fan 94. Thus, the aromatic substance is stably volatilized from when the driving of the fragrance motor 95 starts. Further, the volatilization of the aromatic substance is readily stopped by stopping the fragrance motor 95. Therefore, the adjustment of the volatilization volume of the aromatic substance with the control unit 31a is executed with satisfactory responsiveness as compared to when volatilizing the aromatic substance with a heater, which requires time to raise and lower the temperature.
(8) The air intake port 99 through which the fragrance fan 94 draws in air is formed at the bottom surface of the main body 11. Thus, the fragrance released from the fragrance emission port 15a, which opens in the top surface 12, is not drawn back by the fragrance fan 94. This ensures that the fragrance sent to the user P is satisfactory.
(9) The diameter of the fragrance emission port 15a, the flow rate of air sprayed from the fragrance emission port 15a, and the surface area of the aromatic substance in the fragrance plate 121 are set such that the concentration of the aromatic substance inhaled by the user P in a state mixed with the mists H and C does not exceed a predetermined concentration that may adversely affect the user P. Thus, the user P does not continuously inhale fragrance of which concentration is greater than a level that may adversely affect the user P when using the cosmetic device 10.
(10) The direction changing unit 126 is formed in the fragrance container HY (lid member 123) so that the air conveyed by the fragrance fan 94 flows therein from substantially the entire periphery of the fragrance plate 121, excluding the portion where the hook 120c is arranged. Thus, fresh air is uniformly supplied to the entire contacting surface (surface) of the aromatic substance and the air. This efficiently volatilizes the aromatic substance.
(11) The fragrance plate 121 is fitted to the hook 120c and attached to the ring 122 so as to be fixed to the holder 120. This ensures that the fragrance plate 121 is fixed to the holder 120.
(12) The generation of the mists H and C stops when the fragrance container discharge button 111 is pressed to remove the fragrance container HY from the main body 11 (accommodation chamber 93a). Thus, the user P is prevented from touching the mists H and C when removing the fragrance container HY that is lifted and projected out of upper side of the main body 11 in a state in which the mists H and C are being sprayed from the mist nozzles 18 and 19.
(13) The cap 21 is arranged on the nozzle cover 20 that includes the mist nozzles 18 and 19. This restricts movement of the hand when the user P tries to touch the mist nozzles 18 and 19 and protects the user P.
(14) The cap 21 pivots (moves) from the spray position to the protective position side and covers the upper side of the mist nozzles 18 and 19 when the user P tries to touch the mist nozzles 18 and 19 from above the cap 21. Thus, when the hand of the user P tries to touch the mist nozzles 18 and 19 from above the cap 21, the cap 21 will be located between the mists H and C and the hand of the user P thereby physically preventing the user P from directly touching the mists H and C.
(15) The cap 21 is attached to the nozzle cover 20 (main body 11) in a detachable manner. Thus, the cap 21 may be removed from the mist generator by applying external force. For instance, the cap 21 is removed from the nozzle cover 20 integrally with the nozzle hinge portion 35 when the cosmetic device 10 is dropped or when the user applies excessive force to the cap 21. This prevents damaging of the cap 21. Further, if the cap 21 is damaged, only the cap 21 needs to be replaced, and the main body 11 may be continuously used. This saves resources.
(16) When closing the main body lid 13, the cap 21 moves to the protective position (lower side) as it slides along the slide rib 13c and becomes accommodated in the main body lid 13. The main body lid 13 thus does not need to be large, and the cosmetic device 10 in a state in which the main body lid 13 is closed may be reduced in size. Thus, the cosmetic device 10 may be stored in a small space.
(17) The cap 21 is held at the spray position by the biasing force of the torsion coil spring B2. Thus, when the main body lid 13 opens the cap 21 is returned to the spray position by the biasing force. This prevents the user P from forgetting to attach a cap to the nozzle cover 20 or forgetting to pivot the cap 21 to the spray position. That is, the cap 21 automatically pivots to the protective position and thereby protects the user P.
(18) The fragrance plate 121 is removably attached to the main body 11 while being accommodated in the fragrance container HY. Thus, the fragrance plate 121 holding the aromatic substance does not need to be handled as a single body. This prevents the user P from accidentally touching the aromatic substance. The handling of the fragrance plate 121 is facilitated, and the user P does not drop or knock over the fragrance plate 121. Compared to when the portion for holding the aromatic substance is provided on the main body 11, the task of dropping the aromatic substance is easily carried near by the user, and the aromatic substance is suppressed from being mistakenly dropped to locations other than the interior (recess 121b) of the fragrance plate 121.
(19) The fragrance plate 121 is removably attached to the holder 120 of the fragrance container HY. Thus, the fragrance plate 121 is easily washed by removing it from the holder 120 after use.
(20) The fold-back portion 121a is formed at the opening edge of the fragrance plate 121. Thus, even if the fragrance plate 121 is knocked over, the aromatic substance is held by the fold-back portion 121a and does not flow out.
(21) If the nozzle cover 20 is adjusted to a lower position such that the spraying direction of the mists H and C is closer to the lower limit, the fragrance container HY interferes with (contacts) the mist guide 20b and cannot be removed from the upper side (side of the mists H and C). Thus the user P needs to adjust the nozzle cover 20 to the upper side such that the spraying direction of the mists H and C is closer to the upper limit in order to remove the fragrance container HY. Therefore, the user P adjusts the nozzle cover 20 to the upper side and removes the fragrance container HY after ensuring space for the hand between the mists H and C and the fragrance container HY.
(22) The fragrance generation unit 15 and the warm mist generation mechanism 40 are separately formed. Thus, influences such as the volatilization of the aromatic substance in the fragrance container HY being enhanced by the warm mist heater 41a of the warm mist generation mechanism 40 and the heat of the warm mist are suppressed. Therefore, the blast of the fragrance fan 94 and the adjustment of the volatilization volume by the fragrance plate 121 are facilitated.
(23) The volatilized aromatic substance is fed to the user P in a concentrated manner when mixed with the mists H and C. Thus, compared to when generating the fragrance throughout the entire room, the user senses the fragrance with the volatilization volume of a small amount of aromatic substance. Therefore, the usage amount of the aromatic substance is reduced. This is economical.
(24) The fragrance container HY (fragrance plate 121) for holding the aromatic substance is arranged in the blast passage S2 through which the mists H and C do not pass. Thus, the aromatic substance in the fragrance plate 121 and the mists H and C do not come into direct contact, and the mists H and C do not contain the liquid aromatic substance when sprayed toward the user P. This ensures the safety of the user P.
(25) The fragrance container HY includes the rubber member 125 so as to project out and contact the inner wall surface of the accommodation chamber 93a. Thus, when removing the fragrance container HY from the accommodation chamber 93a, friction occurs between the rubber member 125 and the inner wall surface of the accommodation chamber 93a, and the fragrance container HY is prevented from jumping out of the main body 11 (accommodation chamber 93a) due to the biasing force of the lift spring 104.
(26) The fragrance plate 121 is covered by the lid member 123 and the holder 120, and a space is formed between the fragrance plate 121 and the members 123 and 120 in the fragrance container HY. Thus, the heat of the heaters 41a and 64, which configure the warm mist generation mechanism 40, and the cool mist generation mechanism 60 is less likely to be transmitted to the fragrance plate 121. This prevents volatilization of the aromatic substance in the fragrance plate 121 from being enhanced by the heat of the heaters 41a and 64. The blast of the fragrance fan 94 and the adjustment of the volatilization volume by the fragrance plate 121 are facilitated.
(27) The fragrance emission port 15a is formed to have a grid-shape with a mesh size that makes the liquid aromatic substance difficult to pass. Thus, the user P cannot drop the aromatic substance to the fragrance plate 121 unless the user P removes the fragrance container HY from the main body 11 and remove the lid member 123. In other words, the user P is prevented from dropping the aromatic substance toward the fragrance plate 121 with the fragrance container HY in the main body 11 (accommodation chamber 93a). The aromatic substance is also prevented from jumping out of the fragrance emission port 15a even when the fragrance container HY is knocked over with the aromatic substance in the fragrance plate 121.
(28) The mesh size of the grid in the fragrance emission port 15a is such that the user P would not drop the aromatic substance into the fragrance plate 121 from above the fragrance emission port 15a. Thus, the user P is prevented from dropping the aromatic substance into the fragrance plate 121 when the fragrance container HY is accommodated in the main body 11 (accommodation chamber 93a).

The present invention may be embodied in the following forms.

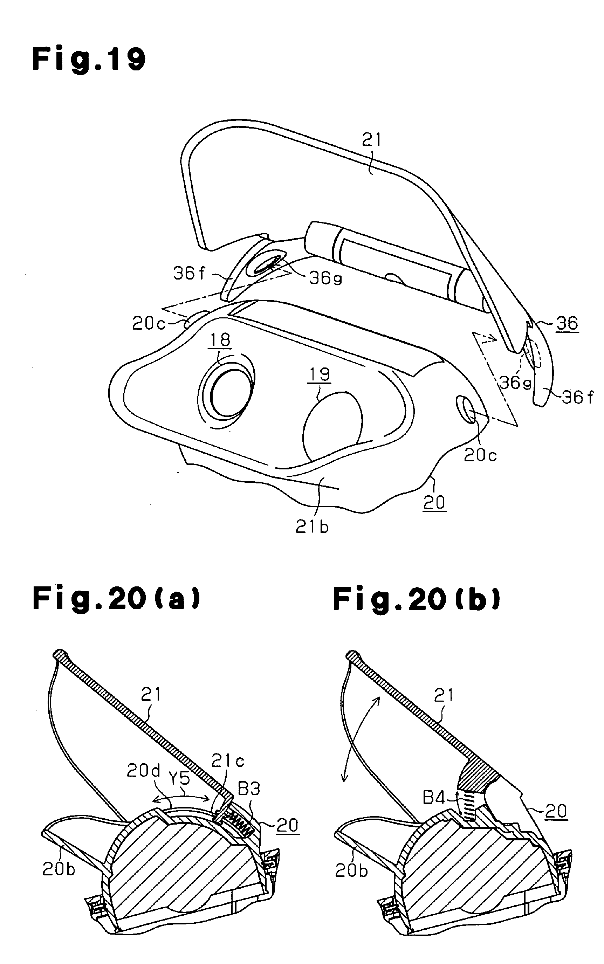

As shown in Fig. 19, a holding portion 36f is extended in a plate-shape from both left and right ends of a nozzle hinge 36 so as to cover the rear side of the nozzle cover 20, and a recess 36g is formed on the inner side of each holding portion 36f. A projection 20c that engages the recess 36g is formed on both side surfaces of the nozzle cover 20 corresponding to each recess 36g. Even with such configuration, the cap 21 may be removably attached to the nozzle cover 20 since the plate-shaped holding portion 36f easily deforms.

Springs other than the torsion coil spring B2 may be used as a means for biasing the cap 21 to maintain the spray position. For instance, as shown in Fig. 20(a), an engagement portion 21c formed in the cap 21 engages a guide 20d formed to extend in the front and back direction at the upper surface of the nozzle cover 20 so that the cap 21 is slidable in the front and back direction (shown with arrow Y5) with respect to the nozzle cover 20. One end of the coil spring B3 may be fixed to the rear part of the cap 21, and the other end may be fixed to the nozzle cover 20 to bias the cap 21 so as to move to the rear side. Furthermore, as shown in Fig. 20(b), a coil spring B4 may be arranged between the cap 21 and the nozzle cover 20 to bias and upwardly move the cap 21. Even with such configuration, the cap 21 can be constantly held at the spraying position by the biasing forces of the coil springs B3 and B4 and can be moved toward the front to the protective position by the hand PH of the user P.

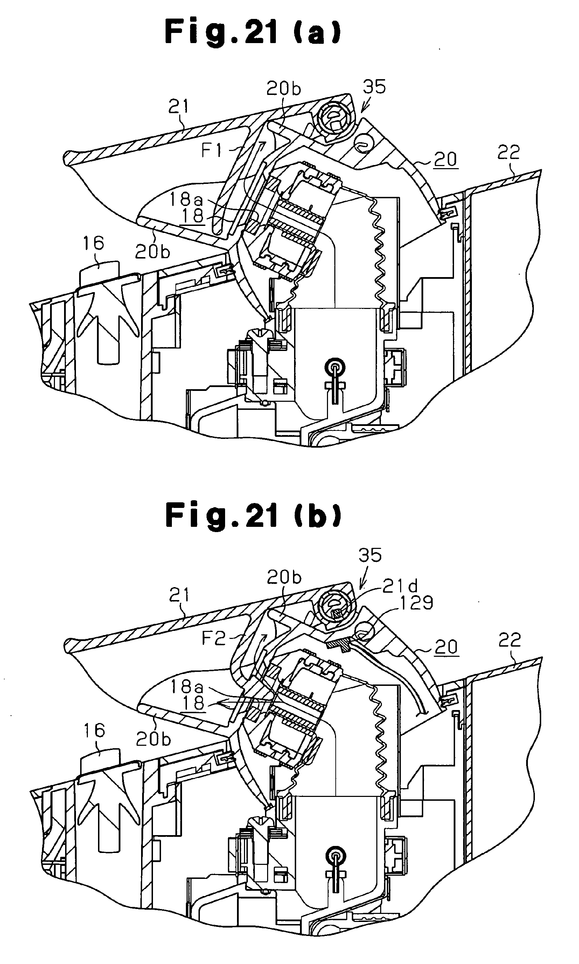

The cap 21 may include a blocking portion for blocking the warm mist H sprayed from the warm mist spray port 18a in cooperation with the movement of the cap 21 from the spray position to the protective position side. Specifically, a wall-shaped blocking portion F1 is formed from the inner side toward the lower side of the cap 21 so as to be arranged parallel to the warm mist spray port 18a and cover substantially the entire surface of the warm mist spray port 18a when the cap 21 is at the protective position, as shown in Fig. 21(a). Furthermore, as shown in Fig. 21(b), a blocking portion F2 having a conical distal end may be inserted into the warm mist spray port 18a when the cap 21 is at the protective position. Each of the blocking portions F1 and F2 do not interfere with the spraying of the warm mist H when the cap 21 is at the spray position. Even with such configuration, the warm mist is blocked by each of the blocking portions F1 and F2, and the warm mist is suppressed from touching the hand PH of the user P at a position close to the warm mist spray port 18a when the cap 21 is at the protective position. The pressure inside the warm mist nozzle 18 rises by blocking the warm mist spray port 18a with the blocking portions F1 and F2. The temperature of the warm mist H may be lowered due to adiabatic expansion when spraying the warm mist H from the gap between the blocking portions F1 and F2 and the warm mist spray port 18a. This further ensures protection of the user P.

The fragrance emission port 15a may be formed by a plurality of circular holes instead of being formed to a grid-shape. In this case, the diameter of each circular hole formed in the fragrance emission port 15a has a size that does not interfere with the spraying of air conveyed by the fragrance fan 94 and has a diameter at which the liquid aromatic substance is held at the circular hole and is difficult to pass therethrough due to surface tension. The diameter of each circular hole of the fragrance emission port 15a may have a size (fineness) that suppresses the user P from trying to drop the aromatic substance to the fragrance plate 121 from above the fragrance emission port 15a. Even with such configuration, the user P is prevented from dropping the aromatic substance toward the fragrance plate 121 while the fragrance container HY is accommodated in the main body 11 (accommodation chamber 93a). The aromatic substance is also suppressed from jumping out even when the fragrance container HY is knocked over.

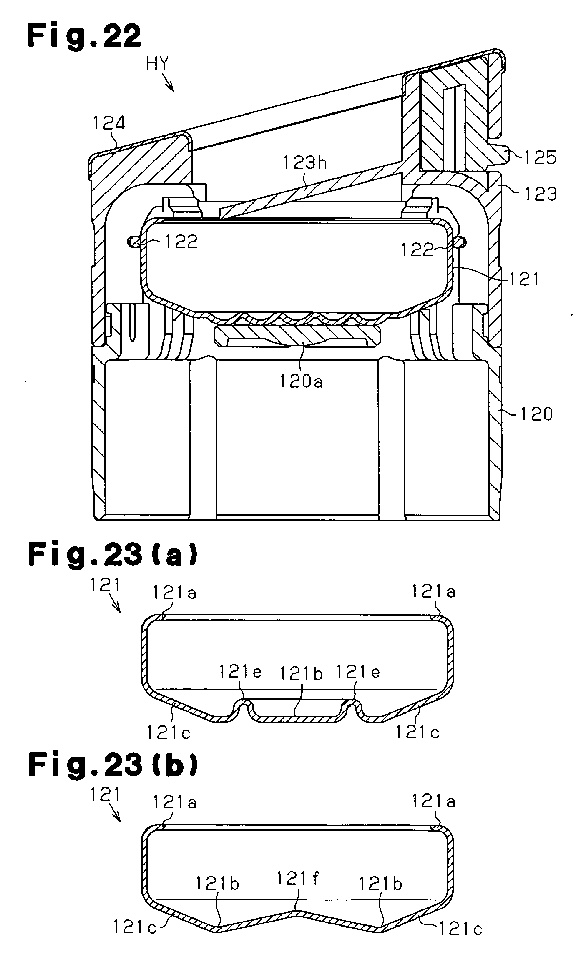

A baffle plate 123h that covers part of the opening of the fragrance plate 121 may be formed inside the lid member 123, as shown in Fig. 22. Even with such configuration, the user P cannot drop the aromatic substance to the fragrance plate 121 unless the user P removes the fragrance container HY from the main body 11 and removes the lid member 123. As the fragrance plate 121 accommodated inside the fragrance container HY cannot be visually recognized from above the fragrance emission port 15a, the user P is prevented from trying to drop the aromatic substance to the fragrance plate 121 from above the fragrance emission port 15a. The aromatic substance is also suppressed from jumping out such as when the fragrance container HY is knocked over.

The recess 121b may have a different shape as long as the surface area of the dropped aromatic substance can be adjusted. As shown in Fig. 23(a), a circular ring-shaped projection wall 121e may be arranged at the bottom of the fragrance plate 121 to form a circular recess 121b. Even with such configuration, the surface area of the aromatic substance dropped into the recess 121b does not become greater than or equal to the area of the part formed with the recess 121b if the dropped amount of aromatic substance is the predetermined amount not exceeding the recess volume. Furthermore, the surface area of the aromatic substance is adjusted by the inclined portion 121c if the aromatic substance of an amount exceeding the recess volume is dropped. Therefore, the volatilization volume of the aromatic substance can be adjusted by adjusting the surface area of the dropped aromatic substance. As shown in Fig. 23(b), the circular ring-shaped recess 121b may be formed at the periphery of the projection 121f by forming the projection 121f projecting to a substantially conical shape at the middle of the bottom of the fragrance plate 121. Even with such configuration, the recess 121b can adjust the surface area of the dropped aromatic substance.

The shape of the recess 121b is not limited to being formed to a semispherical shape, and may be appropriately changed. For example, the recess 121b may be formed as a hexagon without a gap at the bottom of the fragrance plate 121. Even with such configuration, the surface area of the aromatic substance can be adjusted, and the thickness of the wall separating each recess 121b can be thinned.

All controls related to the operation of the cosmetic device 10 do not have to be executed by the control unit 31a, and a fragrance control unit for controlling the fragrance generation unit 15 may be separately arranged.

The generation of the mists H and C may be stopped when detecting movement of the cap 21 to the protective position. Specifically, as shown in Fig. 21(b), a magnet 21d is arranged on the hinge 32, and the lead switch type cap switch 129 is arranged at the corresponding position of the nozzle cover 20. The cap switch 129 is connected to the control unit 31a. The control unit 31a controls the power supply circuit board 30 so as to stop the spraying (generation) of the mists H and C when movement of the cap 21 from the spray position to the protective position is detected, and an on signal output from the cap switch 129 is input. Therefore, when the cap 21 is moved to the protective position, the spraying of the mists H and C is stopped, and the mist H and C do not strike the hand PH of the user P. In this case, the power supply circuit board 30 may be controlled to reduce the spraying amount of the mists H and C. Thus, when the cap 21 is moved to a second position, the warm mist not long after being sprayed from the mist nozzle is suppressed from directly touching the hand of the user. The cap switch 129 may be a detection switch of a mechanical type instead of a lead switch type.

The mist nozzles 18 and 19 may be arranged in a tandem shape in the front and rear directions or the vertical direction in the main body 11. In this case, the fragrance emission port 15a is arranged along a straight line when viewed from the front. Even with such configuration, the fragrance emitted from the fragrance emission port 15a and the mists H and C may be mixed and blended.

Three or more mist nozzles may be used. In this case, the fragrance emission port 15a is arranged between the mist nozzles located at the two sides. Even with such configuration, the fragrance can be emitted toward the mists H and C sprayed from each mist nozzle, and the unevenness in strength in the direction between the mists sprayed from each mist nozzle can be suppressed.

Only one mist nozzle may be used.

The mist sprayed from the mist nozzle may only be either the warm mist H or the cool mist C.

The fragrance emission port 15a may be arranged in the nozzle cover 20 to be positioned between the warm mist nozzle 18 and the cool mist nozzle 19. Even with such configuration, the fragrance emitted from the fragrance emission port 15a and the mists H and C can be mixed and blended.

The fragrance emission port 15a may not be formed in the middle of the warm mist spray port 18a (warm mist nozzle 18) and the cool mist spray port 19a (cool mist nozzle 19). In other words, the fragrance emission port 15a merely needs to be formed between the warm mist spray port 18a (warm mist nozzle 18) and the cool mist spray port 19a (cool mist nozzle 19). Even with such configuration, the fragrance emitted from the fragrance emission port 15a and the mists H and C may be mixed and blended.

The control unit 31a may reduce the intensity (concentration) of the fragrance at predetermined time intervals. Even with such configuration, the olfactory intensity of the user P with respect to fragrance can be recovered and the user P may continuously sense the fragrance.

The flow rate of air sprayed from the fragrance emission port 15a may be changed as required.

The warm mist H and the cool mist C may be simultaneously sprayed. Furthermore, in the present embodiment, the operation of spraying only the cool mist C may be set.

A projection or a plate spring may be arranged on the lid member 123 or the accommodation chamber 93a in place of the rubber member 125. Even with such configuration, the fragrance container HY is prevented from jumping out from the accommodation chamber 93a.

The fragrance plate 121 may be made of ceramic or made of plastic.

The fragrance plate 121 does not have to be made of metal and may be a disposable container made of plastic. According to such configuration, the mixing of fragrance of the aromatic substance by using the same fragrance plate 121 over and over is prevented. Compared to when the fragrance plate 121 is formed integrally with the holder 120, the usage amount of the raw material, the cost, the storage space of the fragrance plate 121, and the environmental load can be reduced.

A plurality of types of fragrance plates 121 having different size and number for the recess 121b and different angle and width for the inclined portion 121c may be prepared, and the fragrance plate may be changed according to the intensity (concentration) of the fragrance for use.

The cooling unit 62 of a water cooling type or Peltier element may be used for cooling in place of air cooling by the heat radiating member 68. Even with such configuration, the water heated by the heating processing unit 61 can be cooled.

The aromatic substance accumulated in the fragrance plate 121 may be volatilized using the heater according to the control of the control unit 31a. In this case, the heater configures the volatilizing means, and the control unit 31a for controlling the operation of the heater configures the adjustment means.

The air intake port 99 may be formed at a location other than the bottom surface of the main body 11. In other words, the air intake port 99 is formed at a location where the fragrance emitted from the fragrance emission port 15a is not drawn in. For instance, the air intake port 99 is formed on the side surface at the rear side of the main body 11.

The fragrance plate 121 may be formed integrally with the holder 120. Even with such configuration, handling is facilitated compared to when handling the fragrance plate 121 as a single body.

The shape of the fragrance plate 121 does not need to be circular and may be polygonal.

The flow of air conveyed by the fragrance fan 94 is not limited to being conveyed from the lower side of the fragrance plate 121, and may be conveyed from the side. In this case, the direction changing unit 126 is configured to change the path such that the air conveyed from the side flows in from substantially the entire periphery of the fragrance plate 121.

The fragrance emission port 15a merely needs to be formed between the mist spray ports 18a and 19a and the shape thereof is not limited to a circle. For instance, the fragrance emission port 15a may be formed to an ellipse, a square, or a polygon. Even with such configuration, the fragrance can be emitted from the fragrance emission port 15a to both the warm mist H and the cool mist C, and the intensity (concentration) of the fragrance can be suppressed from being uneven in each mist H, C.

The liquid (e.g., water) mist generated by ultrasonic wave may be sprayed from the mist nozzle.

## Claims

1. A mist generator (10) comprising:
a warm mist generation mechanism (40) which generates warm mist and includes a warm mist nozzle (18) for spraying the generated warm mist in a spraying direction; and
a movable protective device (21) which prevents a hand (PH) of a user (P) from touching the warm mist nozzle (18), wherein the protective device (21) is movable between a first position, which allows the warm mist to be sprayed from the warm mist nozzle (18) toward the user (P), and a second position, which covers at least an upper part of the warm mist nozzle (18).

2. The mist generator according to claim 1, wherein the protective device (21) is detachable from the mist generator.

3. The mist generator according to claim 1, further comprising:
a main body lid (13) which is openable and closable;
wherein the warm mist nozzle (18) and the protective device (21) are covered by the main body lid (13) in a closed state; and
the protective device moves to the second position in cooperation with the closing of the main body lid (13).

4. The mist generator according to claim 1, wherein the protective device (21) includes a blocking portion (F1; F2), in which the blocking portion (F1; F2) faces toward a spray port (18a) of the warm mist nozzle (18) and blocks a flow of warm mist when the protective device (21) is located at the second position.

5. The mist generator according to claim 1, wherein the protective device (21) is biased to return to the first position from the second position.

6. The mist generator according to claim 1, wherein the warm mist generation mechanism (40) includes:
a heater (41a) which generates warm mist by heating liquid; and
a control unit (31a) which controls current flow to the heater to at least reduce a spraying amount of the warm mist when the protective device (21) is moved from the first position.
